# EUROPEAN PATENT APPLICATION

(11) **EP 4 006 918 A1**
(43) Date of publication of application: **01.06.2022**
(21) Application number: 20848544.1
(22) Date of filing: 25.05.2020
(51) Int. Cl.: G16Z 99/00, G06F 30/10, G06F 30/27

(54) **FINITE FLOW PATTERN WORD REPRESENTATION DEVICE, WORD REPRESENTATION METHOD, AND PROGRAM, STRUCTURE SHAPE LEARNING METHOD, AND STRUCTURE DESIGNING METHOD**

(30) Priority: 30.07.2019 JP 2019139657
(71) Applicant: Japan Science and Technology Agency, Kawaguchi-shi, Saitama 332-0012 (JP)
(72) Inventor: YOKOYAMA, Tomoo, Kyoto-shi Kyoto 612-8522 (JP); SAKAJO, Takashi, Kyoto-shi Kyoto 606-8502 (JP)
(74) Representative: Noble, Nicholas
(86) International application number: PCT/JP2020/020584
(87) International publication number: WO 2021/019883

(57) **Abstract**

A word representation device is provided with a storage, and a word representation generator, in which the storage stores a correspondence relationship between each streamline structure and a character thereof regarding a plurality of streamline structures forming the flow pattern, the word representation generator is provided with a root determination means, a tree representation forming means, and a COT representation generation means, the root determination means determines a root of a given flow pattern, the tree representation forming means forms a tree representation of the given flow pattern by repeatedly executing processing of extracting a streamline structure of the given flow pattern, assigning a character to the extracted streamline structure on the basis of the correspondence relationship stored in the storage, and deleting the extracted streamline structure from an innermost portion of the flow pattern until reaching the root, and the COT representation generation means converts the tree representation formed by the tree representation forming means to a COT representation to generate a word representation of the given flow pattern. A learned AI is allowed to calculate a structure shape that generates a desired flow pattern around the same.

## Description

### [TECHNICAL FIELD]

The present invention relates to a word representation device of a flow pattern, a word representation method, a program, a learning method of a structure shape, and a structure designing method.

### [BACKGROUND ART]

A technology of giving a many-to-one word representation (maximum word representation) to a flow pattern of an incompressible fluid on a curved surface, and a designing method of a shape of a structure in a fluid using the word representation and a regular expression are disclosed (refer to, for example, Patent Literature 1). A technology of acquiring transition information regarding a transition root from a structurally stable streamline pattern in a topological two-dimensional flow structure to another structurally stable streamline pattern that may be taken topologically is disclosed (refer to, for example, Patent Literature 2). Furthermore, a technology of giving a word representation (regular expression) from a graph representation corresponding to a flow pattern on a one-to-one basis to a flow structure of an incompressible fluid on a curved surface, and a technology of optimizing a shape of a structure in a fluid using this are disclosed (refer to, for example, Patent Literature 3).

### [Citation List]

### [Patent Literature]

[Patent Literature 1] WO 2014/041917 A
[Patent Literature 2] WO 2015/068784 A
[Patent Literature 3] WO 2016/072515 A

### [SUMMARY OF INVENTION]

### [TECHNICAL PROBLEM]

The above-described conventional art may give a word representation to a flow pattern of an incompressible fluid on a curved surface, but cannot give a word representation to a general flow pattern including a compressible fluid.

The present invention is achieved in view of such problems, and an object thereof is to give a word representation to a general fluid flow pattern including a compressible fluid. Another object of the present invention is to provide a designing method for giving a shape of a structure such that a flow pattern around a structure present in a general flow including such compressible fluid becomes a desired pattern.

### [SOLUTION TO PROBLEM]

In order to solve the above-described problem, a word representation device according to an aspect of the present invention is a word representation device that performs word representation of a streamline structure of a flow pattern in a two-dimensional domain provided with a storage and a word representation generator. The storage stores a correspondence relationship between each streamline structure and a character thereof regarding a plurality of streamline structures forming a flow pattern, and the word representation generator is provided with a root determination means, a tree representation forming means, and a COT representation generation means. The root determination means determines a root of a given flow pattern, the tree representation forming means forms a tree representation of the given flow pattern by repeatedly executing processing of extracting a streamline structure of the given flow pattern, assigning a character to the extracted streamline structure on the basis of the correspondence relationship stored in the storage, and deleting the extracted streamline structure from an innermost portion of the flow pattern until reaching the root, and the COT representation generation means converts the tree representation formed by the tree representation forming means to a COT representation to generate a word representation of the given flow pattern.

According to this aspect, it is possible to give the word representation to the flow pattern of a general fluid including a compressible fluid by using the device.

Among the streamline structures forming the flow pattern, basic structures may be σ_{ϕ±}, σ_{ϕ~±0}, σ_{ϕ∼±±}, σ_{ϕ~±}∓, β_{ϕ±}, and β_{ϕ2}.

Among the streamline structures forming the flow pattern, two-dimensional structures may be b_{~±} and b_{±}.

Among the streamline structures forming the flow pattern, zero-dimensional point structures may be σ_{∼±0}, σ_{~±±}, and σ_{~±}∓.

Among the streamline structures forming the flow pattern, one-dimensional structures may be p_{~±}, p_{±}, a_{±}, q_{±}, b_{±±}, b_{±}∓, β_{±}, c_{±}, C_{2±}, a₂, γ_{ϕ∼±}, γ_{-±±}, a_{~±} and q_{~±}.

The word representation generator may further be provided with a combinatorial structure extraction means. The combinatorial structure extraction means may generate a word representation having a one-to-one correspondence of a given flow pattern by extracting a combinatorial structure from the given flow pattern.

Another aspect of the present invention is a word representation method. This method is a word representation method of performing word representation of a streamline structure of a flow pattern in a two-dimensional domain executed by a computer provided with a storage and a word representation generator, the storage storing a correspondence relationship between each streamline structure and a character thereof regarding a plurality of streamline structures forming the flow pattern, and the word representation generator executing a root determination step, a tree representation forming step, and a COT representation generation step, in which the root determination step determines a root of a given flow pattern, the tree representation forming step forms a tree representation of the given flow pattern by repeatedly executing processing of extracting a streamline structure of the given flow pattern, assigning a character to the extracted streamline structure on the basis of the correspondence relationship stored in the storage, and deleting the extracted streamline structure from an innermost portion of the flow pattern until reaching the root, and the COT representation generation step converts the tree representation formed of the tree representation forming step to a COT representation to generate a word representation of the given flow pattern.

According to this aspect, it is possible to give the word representation to the flow pattern of a general fluid including a compressible fluid by using the computer.

Still another aspect of the present invention is a program. This program allows a computer provided with a storage and a word representation generator to execute processing. The storage stores a correspondence relationship between each streamline structure and a character thereof for a plurality of streamline structures forming a flow pattern. This program allows a word representation generator to execute a root determination step of determining a root of a given flow pattern, a tree representation forming step of forming a tree representation of the given flow pattern by repeatedly executing processing of extracting a streamline structure of the given flow pattern, assigning a character to the extracted streamline structure on the basis of the correspondence relationship stored in the storage, and deleting the extracted streamline structure from an innermost portion of the flow pattern until reaching the root, and a COT representation generation step of converting the tree representation formed at the tree representation forming step to a COT representation to generate a word representation of the given flow pattern.

According to this aspect, it is possible to implement a program that gives a word representation to a flow pattern of a general fluid including a compressible fluid in a storage medium and the like.

Still another aspect of the present invention is a method. This method is a learning method of learning a shape of a structure in a fluid in a two-dimensional domain, provided with performing word representation of a streamline structure of a flow pattern generated around a structure in a fluid by using the word representation device according to claim 1, and learning by AI a relationship between a three-dimensional shape of the structure and the word representation.

Still another aspect of the present invention is also a method. This method is a structure designing method of designing a structure in a fluid in a two-dimensional domain provided with performing word representation of a streamline structure of a flow pattern generated around a structure in a fluid by using the word representation device according to claim 1, learning a relationship between a three-dimensional shape of the structure and the word representation by AI, performing word representation of a fluid structure of a target flow pattern by using the word representation device according to claim 1, inputting a word representation of the target flow pattern to the AI, and calculating and outputting a three-dimensional shape of a structure that realizes the target flow pattern by the AI.

According to this aspect, an optimum structure shape for controlling the flow of the fluid may be obtained.

Note that arbitrary combination of the above-described components, and mutual substitution of the components and expressions of the present invention among a method, a device, a program, a temporary or non-temporary storage medium recording the program, a system and the like is also effective as the aspect of the present invention.

### [ADVANTAGEOUS EFFECTS OF INVENTION]

According to the present invention, it is possible to give a word representation to a flow pattern of a general fluid including a compressible fluid on a curved surface, and calculate a structure shape that generates a desired flow pattern around the same.

### [BRIEF DESCRIPTION OF DRAWINGS]

Fig. 1 is a diagram illustrating a zero-dimensional point structure. Fig. 1A illustrates a center. Fig. 1B illustrates a saddle. Fig. 1C illustrates a ∂-saddle. Fig. 1D illustrates a source. Fig. 1E illustrates a ∂-source. Fig. 1F illustrates a sink. Fig. 1G illustrates a ∂-sink.
Fig. 2 is a diagram illustrating a circuit as a one-dimensional structure. Fig. 2A illustrates a cycle. Fig. 2B illustrates an orbit.
Fig. 3 is a diagram illustrating a saddle separatrix as a one-dimensional structure. Fig. 3A illustrates an orbit of a self-connected saddle separatrix. Fig. 3B illustrates an orbit of a heteroclinic saddle separatrix.
Figs. 4A and 4B are diagrams illustrating ss-components as one-dimensional structures and an ss-separatrix connecting them.
Fig. 5 is a diagram illustrating a slidable saddle and a slidable ∂-saddle as one-dimensional structures. Fig. 5A and Fig. 5B illustrate a slidable saddle. Fig. 5C and Fig. 5D illustrate a slidable ∂-saddle.
Fig. 6 is a diagram illustrating a two-dimensional structure. Fig. 6A illustrate a trivial center disk. Fig. 6B illustrates a trivial source disk. Fig. 6C illustrates a periodic border annulus. Fig. 6D illustrates a limit annulus. Fig. 6E illustrates a periodic annulus. Fig. 6F illustrates a rotating sphere.
Fig. 7 is a diagram illustrating a Reeb domain and a rotating annulus as two-dimensional structures. Fig. 7A and Fig. 7B illustrate a Reeb domain. Fig. 7C illustrates a rotating annulus.
Fig. 8 is a diagram illustrating four types of non-trivial limit circuits appearing in a flow of finite type.
Fig. 9 is a diagram illustrating a classification of orbit groups in a domain appearing in a complementary set of a set Bd(v) of border orbits of the flow of finite type. Fig. 9A illustrates an open rectangular domain in which an orbit space is an open interval. Fig. 9B illustrates an open annular domain in which an orbit space is a circle. Fig. 9C illustrates an open annular domain in which an orbit space is an open interval.
Fig. 10 is a drawing illustrating a basic streamline structure of a structurally stable Hamiltonian vector field. Fig. 10A illustrates a uniform flow in an unbounded domain. Fig. 10B illustrates a counterclockwise flow in a bounded domain. Fig. 10C illustrates a clockwise flow in the bounded domain.
Fig. 11 is a diagram illustrating a local streamline structure that enters the structurally stable Hamiltonian vector field. Fig. 11A illustrates a class-a streamline structure. Fig. 11B illustrates a class-b streamline structure. Fig. 11C illustrates a class-c streamline structure.
Fig. 12 is a diagram illustrating an example of a flow pattern to be characterized.
Fig. 13 is a diagram illustrating a structure (two-dimensional structure) representing an orbit group of (Bd(v))^{c}. Fig. 13A illustrates a structure b_{~±}. Fig. 13B illustrates a structure b_{±}. A structure is not assigned to Fig. 13C being an obvious structure.
Fig. 14 is a diagram illustrating a basic structure on a spherical surface S. Fig. 14A illustrates structures σ_{ϕ±}, σ_{ϕ∼±0}, σ_{ϕ∼±±}, and σ_{ϕ~±}∓. Fig. 14B illustrates a structure β_{ϕ±}. Fig. 14C illustrates a structure β_{ϕ2}.
Fig. 15 is a diagram illustrating an example of a zero-dimensional point structure and a one-dimensional structure of Bd(v). Fig. 15A illustrates a structure β_{±} as the one-dimensional structure. Fig. 15B illustrates structures σ_{±}, σ_{∼±0}, σ_{~±±}, and σ_{~±}∓ as the zero-dimensional point structures. Fig. 15C illustrates a structure p_{~±} as the one-dimensional structure. Fig. 15D illustrates a structure p_{±} as the one-dimensional structure.
Fig. 16 illustrates a (S1)-series one-dimensional structure. Fig. 16A illustrates a structure a_{±}. Fig. 16B illustrates a structure q_{±}.
Fig. 17 is a diagram illustrating a (S2)-series one-dimensional structure. Fig. 17A illustrates a structure b_{±±}. Fig. 17B illustrates a structure b_{±}∓.
Fig. 18 is a diagram illustrating a (S4)-series one-dimensional structure. Fig. 18A illustrates a structure β_{±}. Fig. 18B illustrates a structure c_{±}. Fig. 18C illustrates a structure c_{2±}.
Fig. 19 is a diagram illustrating a (S5)-series one-dimensional structure. Fig. 19A illustrates a structure a₂. Fig. 19B illustrates a structure γ_{ϕ∼±}. Fig. 19C illustrates a structure γ_{∼±-}. Fig. 19D illustrates a structure γ_{-±+}.
Fig. 20 is a diagram illustrating a (S3)-series one-dimensional structure. Fig. 20A illustrates a structure a_{~±}. Fig. 20B illustrates a structure q_{~±}.
Fig. 21 is an explanatory diagram of a tree representation.
Fig. 22 is a functional block diagram of a word representation device according to a first embodiment.
Fig. 23A, 23B, 23C and 23D are flowcharts of processing of forming a tree representation according to the first embodiment.
Fig. 24A, 24B, and 24C are diagrams illustrating examples of flow patterns on curved surfaces.
Fig. 25A and 25B are diagrams illustrating other examples of flow patterns on curved surfaces.
Fig. 26 is a functional block diagram of a word representation device according to a second embodiment.
Fig. 27A and 27B are diagrams illustrating examples of flow patterns having the same COT representation but different streamline topologies.
Fig. 28 is a diagram illustrating a procedure of extracting a combinatorial structure from the flow pattern in Fig. 27.
Fig. 29 is a diagram illustrating a combinatorial structure to be extracted from the flow pattern.
Fig. 30 is a flowchart illustrating a word representation method according to a third embodiment.
Fig. 31 is a diagram illustrating a flow pattern obtained by simulation from a topographic map of a river and a COT representation thereof.
Fig. 32 is a flowchart illustrating a learning method of a structure shape according to a fifth embodiment.
Fig. 33 is a flowchart illustrating a structure designing method according to a sixth embodiment.
Fig. 34 is a diagram illustrating a desired flow pattern drawn by a user for the topographic diagram in Fig. 31 and a COT representation thereof.

### [DESCRIPTION OF EMBODIMENTS]

The present invention is hereinafter described on the basis of preferred embodiments with reference to each drawing. In the embodiments and variations, the same or equivalent components and members are assigned with the same reference signs, and description is not repeated appropriately. A dimension of the member in each drawing is appropriately scaled in order to facilitate understanding. Some elements not important for illustrating the embodiments in each drawing are not illustrated. Terms including ordinal numbers such as first and second are used for describing various components, but the terms are used only for the purpose of distinguishing one component from other components, and the components are not limited by the terms.

### Overview

In a classification theory of a structurally stable Hamiltonian vector field (incompressible vector field) disclosed in Patent Literature 1, an adjacency relationship of domains divided by five types of characteristic one-dimensional streamline structures has been graphed to obtain character information. In order to expand this to a compressible fluid, in the following embodiments, 14 types of one-dimensional structures are newly added to the above-described five types, and three types of classifications corresponding to orbit groups that fill two-dimensional domains divided by them are also characterized. That is, it is confirmed whether there is the characteristic one-dimensional streamline structure that divides these two-dimensional domains, and if there is, a character corresponding to this is assigned. Furthermore, an entire streamline structure may be represented as a character string by sequentially assigning a character string reflecting information of a streamline group in the same. In an algorithm herein used, the streamline structure is extracted from an innermost portion (fine structure) in a given streamline group, and a character is assigned thereto, then the streamline structures on an outer side (large structures) are sequentially extracted to achieve final characterization. Since the structure cannot be uniquely represented only by the character string due to the expansion to the compressible fluid, an algorithm that combinatorially specifies a connection of the streamline structures is added to obtain a unique representation.

In a regular expression of a structurally stable Hamiltonian vector field disclosed in Patent Literature 3, a structure (uniform flow or periodic orbit) of a streamline group inside thereof has been automatically determined from a one-dimensional structure dividing these domains. Therefore, for the regular expression of information of the streamline group inside, it has been sufficient to simply give a symbol representation of +, -, and o. Since there has been no flow corresponding to a compressed component other than the uniform flow (a pair structure of sink and source at a point of infinity (1-source-sink point)), it has not been necessary to represent a connection. In a case where an algorithm used in the following embodiments is applied to the structurally stable Hamiltonian vector field, it is possible to automatically convert into the regular expression by deleting a structure representing the streamline group inside from the character obtained by the algorithm. Therefore, it may be said that this embodiment includes a technology for the incompressible flow disclosed in Patent Literature 3.

### Definition of Terms

Hereinafter, mathematical terms used in this specification are described. In the following embodiments, a flow subjected to a word representation is a "flow v on a curved surface S". Herein, the "curved surface S" refers to a two-dimensional structure, that is, a two-dimensional compact manifold (herein, especially, a spherical surface that allows presence of a boundary). The spherical surface may be geometrically identified with a plane with a point of infinity added, so that this may be essentially considered as a bounded two-dimensional domain. The "flow v on the curved surface S" refers to an R-action on the curved surface S denoted by v : R × S → S. By using this flow v, for t e R, a mapping vₜ := S → S on S is defined by vₜ := v(t, ·). Herein, for a point x on S, O(x) = {vt(x) ∈ S|t ∈ R} is referred to as an "orbit passing through x". The algorithm of this embodiment classifies topological structures of a set of O(x) on S. The flow v herein defined is a completely abstract target. However, according to a hydrodynamic analogy, these orbit groups correspond to orbit (streamline) groups through which particles are allowed to flow by a given flow field, so that unless there especially is confusion, they are hereinafter referred to as "orbit groups" or "streamline groups".

According to a general mathematical theory (a foliation theory in a field of topology), it is known that orbits of the flow v on the curved surface S are classified into three types of:
(1) proper,
(2) locally dense, and
(3) exceptional.

For this, the following assumption is made in this specification.

### (Assumption) "An orbit set includes only proper orbits."

Note that this assumption is satisfied by "a spherical surface that allows presence of a boundary" targeted by the embodiments of the present invention, so that this is not an essential limitation. The proper orbit set is further classified into three types of orbit groups, that is:
(i) singular orbits,
(ii) periodic orbits, and
(iii) non-closed orbits.

The mathematical definition of them is hereinafter given.

(Definition 1) "For the proper orbits defined by the flow v on the curved surface S,
(i) singular orbits,
(ii) periodic orbits, and
(iii) non-closed orbits
   are hereinafter defined.
   (i) A fact that x e S is a singular orbit means that x = vₜ(x) holds for any t e R, that is, O(x) = {x}.
   (ii) A fact that an orbit O(x) is periodic means that a certain T > 0 exists such that v_{T}(x) = x, and vₜ(x) ≠ x for 0 < t < T.
   (iii) An orbit that is neither the singular orbit nor the periodic orbit is referred to as a non-closed orbit."

The term "non-closed orbit" in (iii) is derived from a fact that the singular orbit and the periodic orbit are a closed set (closed) as the orbit. For future convenience, a set of singular orbits, a set of periodic orbits, and a set of non-closed orbits of the flow v are denoted by Sing(v), Per(v), and P(v), respectively.

Next, several mathematical definitions are provided for describing the streamline structure of the flow. Note that a symbol with macron (- ) above A denotes a closure of a set A.

(Definition 2) For the proper orbit passing through x e S defined by the flow v on the curved surface S, a ω-limit set ω(x) and an α-limit set α(x) are defined as follows.

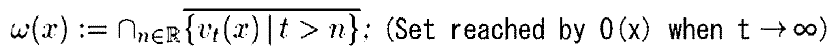

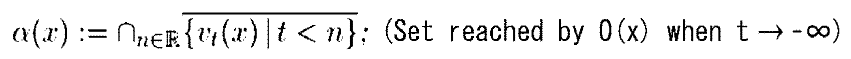

When ω(γ) and α(γ) are the singular orbits, a separatrix γ is connecting.

A definition of the separatrix is described later. Note that, since ω(x) and α(x) do not depend on how to take the point x on the orbit O(x), they are sometimes denoted by ω(O) and α(O), respectively, as symbols.

Next, a flow structure required for classifying streamline groups of a compressible flow is introduced, and described for each of a zero-dimensional point structure, a one-dimensional structure, and a two-dimensional structure.

### Zero-Dimensional Point Structure

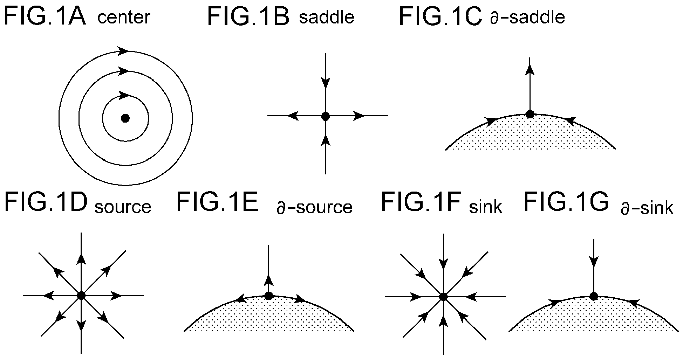

Fig. 1 illustrates all regular zero-dimensional point structures. Fig. 1(a) illustrates a "center". The center represents a singular orbit accompanied with periodic orbits around the same. Fig. 1(b) illustrates a "saddle". The saddle is connected to two separatrices separating from the same and two separatrices approaching the same. Fig. 1(c) illustrates a "∂-saddle" attached to a boundary. At the ∂-saddle, one separatrix extends from the boundary to the inside of S, and two separatrices have orbits along the boundary. Fig. 1(d) illustrates a "source". At the source, a fluid springs from one point. Fig. 1(e) illustrates a "∂-source". At the ∂-source, the source is on a boundary. Fig. 1(f) illustrates a "sink". The sink is obtained by reversing a direction of the source. Fig. 1(g) illustrates a "∂-sink". The ∂-sink is obtained by reversing a direction of the ∂source.

The point structures illustrated in Figs. 1(a) to 1(c) are also observed in the incompressible flow, and are treated in a word representation theory disclosed in Patent Literature 1 and a regular expression theory disclosed in Patent Literature 3. On the other hand, the point structures illustrated in Figs. 1(d) to 1(g) are newly added by the present inventors as this embodiment treats the compressible flow.

### One-Dimensional Structure

### Circuit

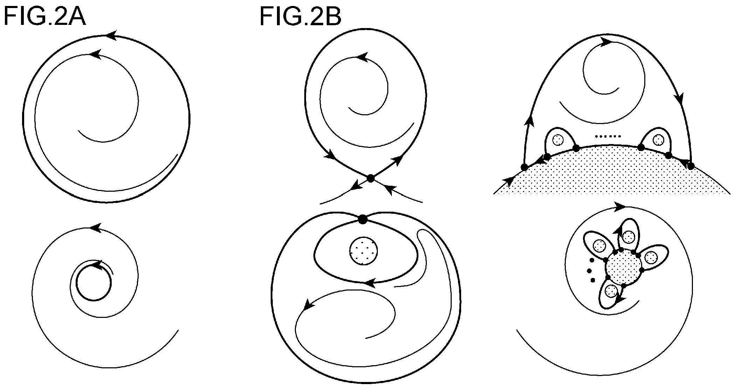

Fig. 2 illustrates a "circuit" as a one-dimensional structure. The "circuit" means an "immersion" of a singleton or a circle onto S. When the circuit is a point, this is referred to as a "trivial circuit", and when the circuit is a circle, this is referred to as a "non-trivial circuit". Furthermore, the non-trivial circuits are classified into two types according to a flow defined thereon. One is when the circle becomes a periodic orbit, which is referred to as a "cycle". The cycle is an element of Per(v). Fig. 2(a) illustrates an example of the cycle. The other is an "orbit" formed of the saddle (an element of Sing(v)) and the separatrix (an element of P(v)) connecting the same. Fig. 2(b) illustrates some examples of the orbit.

Several concepts are defined in association with the circuit. A circuit γ is "attracting" when there is a one-side neighborhood A of the circuit (referred to as an attracting basin of γ) such that one boundary is γ and A ⊆ W^{s}(γ). On the other hand, the circuit γ is "repelling" when there is a one-side neighborhood A of the circuit (referred to as a repelling basin of γ) such that one boundary is γ and A ⊆ W^{u}(γ). Herein, W^{s}(γ) and W^{u}(γ) represent a stable manifold and an unstable manifold of y, respectively. By using this concept, it may be understood that the source and the ∂-source are repelling trivial circuits, and the sink and the ∂-sink are attracting trivial circuits. The orbit γ is a "limit circuit" when this is the non-trivial circuit that satisfies α(x) = γ or ω(x) = γ at a certain point x (not included in γ). As may be understood from this definition, in a case where there is only a finite number of singular orbits, the limit circuit has at least one attracting/repelling basin. Fig. 2 illustrates some examples of the limit circuits.

### (Ss)-saddle Separatrix Structure

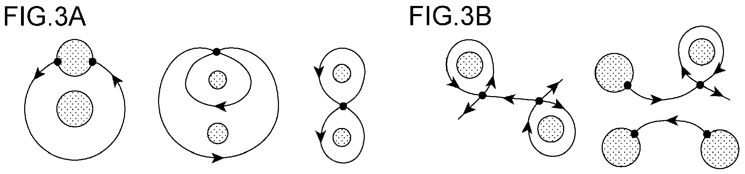

Fig. 3 illustrates a "saddle separatrix (saddle separatrix structure)". The saddle separatrix refers to an orbit the α-limit set and the ω-limitset of which are the saddles or the ∂-saddles. The saddle separatrix is an element of P(v).

When the saddle separatrix connects the same saddle, this is referred to as a "self-connected saddle separatrix". When the saddle separatrix connects two different ∂-saddles on the same boundary, this is referred to as a "self-connected ∂-saddle separatrix". Fig. 3(a) illustrates an example of an orbit of the self-connected saddle separatrix. The saddle separatrix connecting different saddles or ∂-saddles on different boundaries is referred to as a "heteroclinic saddle separatrix". Fig. 3(b) illustrates an example of an orbit of the heteroclinic saddle separatrix.

It is proved that only a self-connected saddle separatrix appears when structural stability is assumed for the Hamiltonian vector field. A "saddle connection diagram" is a set of the saddles, the ∂-saddles, and the saddle separatrices as a whole, which is treated in the word representation theory or the regular expression theory in the conventional art.

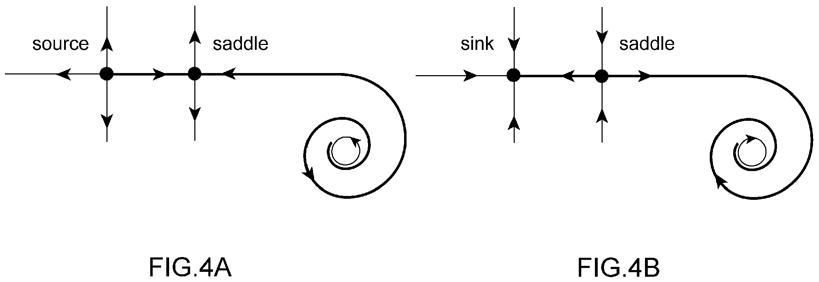

Next, the saddle separatrix specific to a compressible flow structure is defined. An "ss-component" refers to either (1) the (∂-)source, (2) the (∂-)sink, and (3) the non-trivial limit circuit. The separatrix connecting the saddle or ∂-saddle to the ss-component is referred to as an "ss-separatrix". Fig. 4 illustrates an example of the ss-components and the ss-separatrix connecting them. A set of the saddle, ∂-saddle, saddle separatrix, ss-component, and ss-separatrix is referred to as an "ss-saddle connection diagram". Hereinafter, the ss-saddle connection diagram generated by the flow v on the curved surface S is denoted by Dₛₛ(v).

### Slidable (∂-)saddle Structure

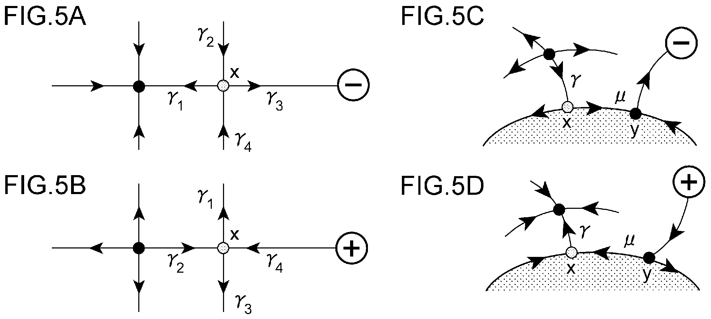

Fig. 5 illustrates a "slidable saddle" and a "slidable ∂-saddle" as one-dimensional structures.

Figs. 5(a) and 5(b) illustrate the point x of the slidable saddle. The saddle x is connected to four separatrices by definition. When they are denoted by γ₁, γ₂, γ₃, and γ₄, α(γ₁) = α(γ₃) = ω(γ₂) = ω(γ₄) = x is satisfied. The "saddle x is slidable" either in a case where "ω(γ₁) is the sink and ω(γ₃) is a sink structure (represented by a symbol in which - is enclosed by ∘ in the drawing)" (Fig. 5(a)) or in a case where "α(γ₂) is the source and α(γ₄) is a source structure (represented by a symbol in which + is enclosed by o in the drawing)" (Fig. 5(b)).

Figs. 5(c) and 5(d) illustrate the point x of the slidable ∂-saddle. The "∂-saddle x is slidable (or x is the slidable ∂-saddle)" when a structure is such that there is a separatrix γ ⊂ intS, a separatrix µ along the boundary, and a ∂-saddle y ≠ x on the same boundary as one x and connected to the sink structure, in which ω(γ) = x, α(γ) is the source, α(µ) = x, and ω(µ) = y (Fig. 5(c)). A structure obtained by reversing directions of vectors is also the slidable ∂-saddle (Fig. 5(d)).

Note that a term "source/sink structure" herein introduced represents a connection destination of the ss-separatrix from the (∂-)saddle, allowing various sink/source structures, such as the source/sink/limit cycle/limit circuit and the like. On the other hand, the other ss-separatrix in the definition of the slidable ∂-saddle allows only the point structure of source/sink, and, in a case where this is referred to as a slidable saddle structure, this represents a set of the saddle x and the ss-separatrix and the source (sink) connected thereto. In a case where this is referred to as a slidable (∂-)saddle structure, this represents a set of two ∂-saddles of x and y, and the ss-separatrix γ and source/sink connected to x.

### Two-Dimensional Structure

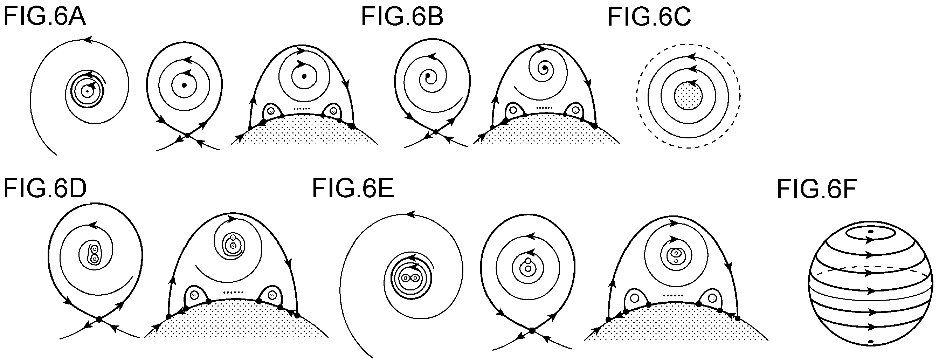

Some two-dimensional structures are illustrated in Fig. 6.

A "center disk" refers to a structure including one center and periodic orbits that fill a periphery thereof. When a boundary of the center disk is any of the limit cycle, the saddle and the self-connected saddle separatrix connecting the same, and two separatrices connecting two ∂saddles on the same boundary, this is referred to as a "trivial center disk". Fig. 6(a) illustrates an example of the trivial center disk.

A "sink (source) disk" refers to a structure formed of one sink (source) and non-closed orbits that fill a periphery thereof. The sink/source disk having no common portion with the separatrix is referred to as a "trivial sink/source disk". Fig. 6(b) illustrates an example of the trivial source disk.

A "periodic border annulus" refers to a structure formed of a boundary and periodic orbits filling a periphery thereof. Especially, the periodic border annulus is a type of a periodic annulus. Fig. 6(c) illustrates an example of the periodic border annulus.

A "limit annulus" refers to an open annular domain filled with non-closed orbits of P(v), a structure in which a boundary is the limit circuit. Fig. 6(d) illustrates an example of the limit annulus.

The "periodic annulus" is an open annular domain filled with periodic orbits of Per(v). Fig. 6(e) illustrates an example of the periodic annulus. Note that, in a case where one boundary of the periodic annulus is the cycle (the non-trivial circuit of Per(v)), this should be the limit cycle on the other side. This is because, if the other side is also filled with periodic orbits s, this cycle cannot be distinguished from normal periodic orbits.

A "rotating sphere" refers to a structure formed of two centers and periodic orbits filling a space therebetween in a flow on an entire spherical surface. This represents a basic flow on an unbounded two-dimensional spherical surface. Fig. 6(f) illustrates an example of the rotating sphere.

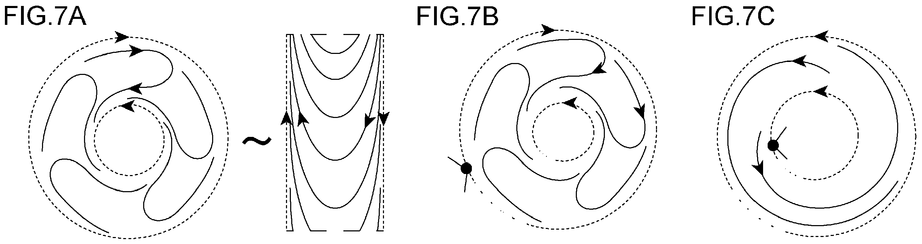

Fig. 7 illustrates a Reeb domain and a rotating annulus as two-dimensional structures.

### Reeb Domain

The "Reeb domain" refers to an open annular domain U filled with the non-closed orbits with two limit circuits γ₊ and γ- present on the boundary thereof, U being a connected component of W^{u}(γ₋) ∩ W^{s}(γ₊), the same structures with rotational directions of γ_{±} reversed. Figs. 7(a) and 7(b) illustrate examples of the Reeb domain. In the Reeb domain in Fig. 7(a), the annular domain is the annular domain filled with the non-closed orbits with flow directions reversed between an inner boundary and an outer boundary. A bilateral boundary is the limit cycle. In the Reeb domain in Fig. 7(b), inner and outer boundaries are the saddle and the self-connected separatrix connecting the same (limit circuits).

### Rotating Annulus

On the other hand, a structure in which the rotational directions of γ_{±} are the same is referred to as a "rotating annulus". Since such a structure is allowed, in a case where the open annular domain is filled with the non-closed orbits, directions of the orbits on the outer and inner boundaries may be independently determined. Fig. 7(c) illustrates an example of the rotating annulus. In this example, as is the case with the Reeb domain, the inside is filled with the non-closed orbits, but flow directions are the same on the outer and inner boundaries.

This is the end of the description of the flow structure in each dimension.

### Phase Classification Theory of Streamline Group of Flow v on Curved Surface S

As a result of intensive studies, the present inventors give a theoretical classification of the orbit groups generated by the flow v on the spherical surface S under the following conditions.

(Definition 3) "When the flow v on the curved surface S satisfies following five conditions, this is referred to as a "flow of finite type".
(1) All orbits generated by v are proper.
(2) All singular orbits are non-degenerate.
(3) The number of limit cycles is finite.
(4) All saddle separatrices connecting the saddles and ∂-saddles are self-connected."

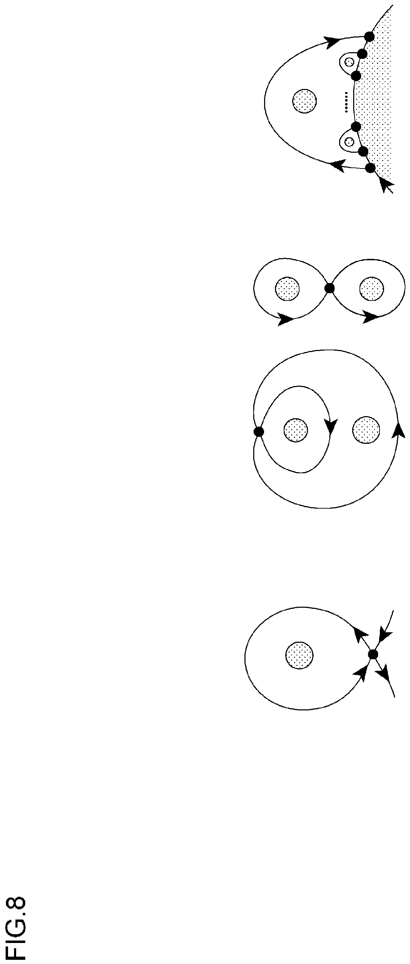

The condition (1) is already assumed. It may be understood that the number of singular orbits is finite and isolated with the condition (2). The condition (3) means that the structures such as limit cycles do not accumulate infinitely. It may also be concluded that there are only four patterns illustrated in Fig. 8 of the non-trivial limit circuits formed of the separatrix connecting the saddles on the basis of the condition (4). The classification theory of the Hamiltonian vector field of existing studies has shown that the conditions (1), (2), and (4) are satisfied by adding a mathematical limitation of structural stability to the flow. In this embodiment, since the condition of incompressibility is removed, the condition of structure stability cannot be imposed. However, in the streamline created by the compressible flow obtained by application, a structure having a degenerate singular orbit, the limit cycles infinitely accumulated, and a heteroclinic orbit is not observed generically due to observation noise, a simulation error and the like. Therefore, the classification theory in this embodiment and an application range of an algorithm based on this theory are not strongly limited.

The classification theory of the flow of finite type is a generalization of Poincare-Bendixon's theorem in a form including a global connection situation. First, the following holds regarding the ω-limitset.

(Lemma 1) "The flow v on the curved surface S is made a flow of finite type. At that time, the ω-limitset (a-limit set) of proper non-closed orbits of v is formed of followings:
(1) saddle,
(2) ∂-saddle,
(3) sink,
(4) source,
(5) ∂-sink,
(6) ∂-source,
(7) center,
(8) attracting (repelling) limit cycle, and
(9) attracting (repelling) non-trivial limit circuit

In the embodiment of the present invention, it is assumed that (5) and (6) do not exist. Actually, this is not a strong constraint because the ∂-sink and ∂-source are not observed generically due to observation noise, simulation error and the like. Although it is possible to remove this condition, local structures increase eventually, and a representation becomes complicated.

According to lemma 1, it may be understood that the orbit groups of the flow of finite type may be classified into following three categories.
(i) The limit sets and the non-closed proper orbits connecting them
(ii) The center, the cycle on a boundary ∂S of S, or the circuit and periodic orbits around the same
(iii) A non-closed orbit group in intP(v)

Next, the orbit groups are classified on the basis of this. A set of structures of one-dimension or less forming the ss-saddle connection diagram Dₛₛ(v) of the flow of finite type v and the boundary ∂S of S are referred to as "border orbits", and are characterized as follows.

(Definition 4) "A set Bd(v) of border orbits of the flow of finite type v on the curved surface S is given as follows: $Bd \left(v\right) : = Sing \left(v\right) ∪ \partial Per \left(v\right) ∪ \partial P \left(v\right) ∪ {P}_{sep} \left(v\right) ∪ {\partial }_{Per} \left(v\right) ,$ wherein each set is an orbit set given below:
(1) Pₛₑₚ(v): an orbit set including a saddle separatrix and an ss-separatrix in an inner point set of P(v),
(2) ∂Per(v): a set of orbits serving as a boundary of a set of periodic orbits,
(3) ∂P(v): a set of orbits serving as a boundary of a set of non-closed orbits, and
(4) ∂_{Per}(v): a set of periodic orbits (∂S ∩ intPer(v)) rotating along the boundary ∂S of S."

Note that, since Pₛₑₚ(v) is the set including the saddle separatrix and ss-separatrix and is a structure at an inner point of the proper orbit, a neighborhood of the structure is also the proper orbit, and also indicates other than a limit set. Note that the orbits of ∂P(v) and ∂Per(v) indicate the limit cycle or the non-trivial limit circuit. In the regular expression theory of the streamline phase structure of the structurally stable Hamiltonian vector field of the conventional studies, an adjacency relationship of domains divided by this border orbit (mathematically represented as (Bd(v))^{c} = S - Bd(v)) has been represented as a graph, and a character string has been assigned thereto. At that time, since the orbit group included in the divided domains is uniquely determined from an incompressibility condition, further information has not been required. On the other hand, in a case of the compressible flow, since there are types of orbit groups in the divided two-dimensional domains, it is necessary to also characterize information thereof. In order to give this classification of the inner orbits, a concept of an "orbit space" obtained by introducing a kind of equivalence relationship into the orbit group is introduced.

(Definition 5) "A proper orbit group generated by the flow v on the curved surface S passes through the inside of an open subset T (c S). At that time, an orbit space T/~ of T is a quotient set introduced from a following equivalence relationship "if arbitrary x, y e T, and O(x) = O(y), then x~y".

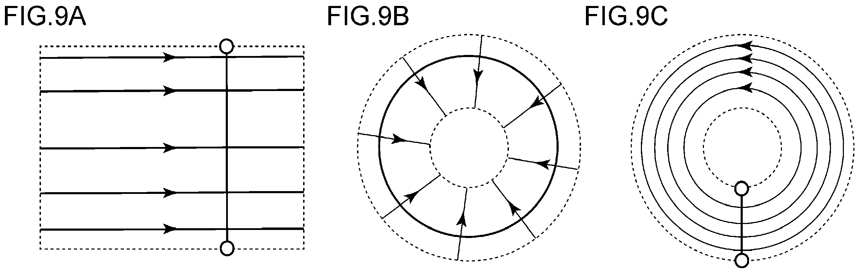

The quotient set in the definition 5 means an operation of collapsing points on the same orbit into one point and identifying them. Fig. 9 illustrates a classification of orbit groups in a domain appearing in a complementary set of the set Bd(v) of border orbits of the flow of finite type v. Fig. 9(a) illustrates an open rectangular domain in which the orbit space is an open interval. That is, in a case where there are flows such as uniform flows in a rectangular open set T as illustrated in Fig. 9(a) in parallel, the orbit space is the open interval. Fig. 9(b) illustrates an open annular domain in which the orbit space is a circle. Fig. 9(c) illustrates an open annular domain in which an orbit space is an open interval. As illustrated in Figs. 9(b) and 9(c), the orbit space of the orbit group in the open annular domain includes the circle and the open interval.

Theorem 1 below claims that "there are only three types illustrated in Fig. 9 of the orbit groups that fill the open domains divided by Bd(v)". The proof of this theorem is given by the present inventor.

(Theorem 1) "For an arbitrary flow of finite type v on a curved surface S ⊆ S², the orbit of the domain in a complementary set (Bd(v))^{c} of a boundary set is any one of following three types.
(1) The open rectangular domain filled with the non-closed orbits of P(v). The orbit space thereof is the open interval (Fig. 9(a)). This is the flow of a neighborhood domain of the ss-separatrix.
(2) The open annular domain filled with the non-closed orbits of P(v). The orbit space thereof is the circle ((Fig. 9(b)). This is a flow of the neighborhood domain of source (sink) disk/limit annulus.
(3) The open annular domain filled with the periodic orbits of intPer(v). The orbit space thereof is the open interval ((Fig. 9(c)). This is the flow of the neighborhood domain of center disk/periodic annulus/rotating sphere."

### COT Representation

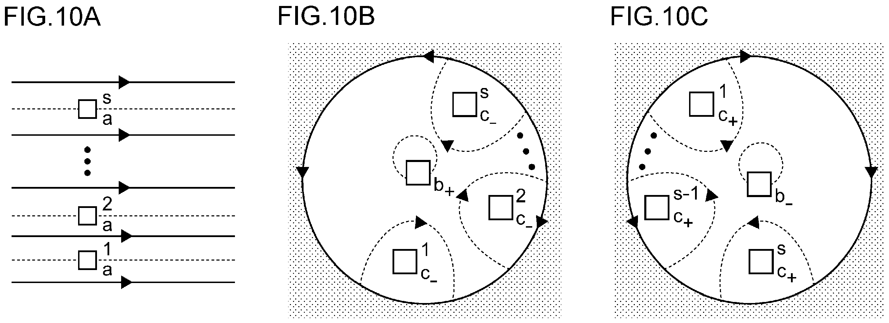

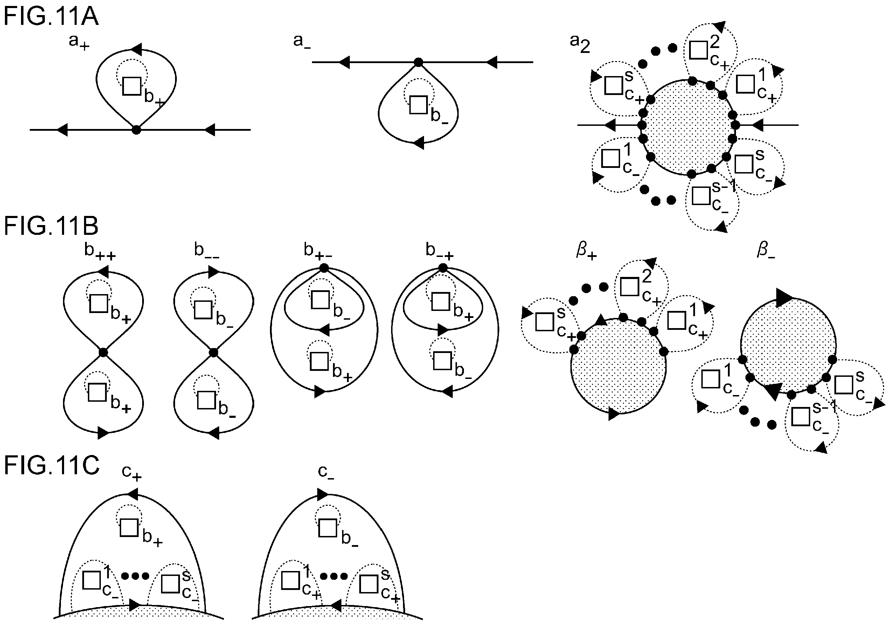

An algorithm used in the embodiment to be described later converts a phase structure of the orbit group of the compressible flow into a "partially cyclically ordered rooted tree representation" (hereinafter, referred to as a "COT representation"). The COT representation is newly devised by the present inventors from a viewpoint of computer science as a data structure for implementing a characterization of the streamline phase structure of the incompressible flow in a computer program. The word representation theory (refer to, for example, Patent Literature 1) and the regular expression theory (refer to, for example, Patent Literature 3), which are conventional studies, are correct as theories, but are somewhat ambiguous from the viewpoint of the data structure for programming as described later. This ambiguity may be completely eliminated by adopting the COT representation. Hereinafter, the COT representation representing the streamline structure of the structurally stable Hamiltonian vector field is described with reference to Figs. 10 and 11. Note that the COT representation of the flow of finite type described in this specification is an extension of the representations illustrated in Figs. 10 and 11 in a natural manner so as to be able to cope with the compressible flow.

Fig. 10 illustrates a basic streamline structure of the structurally stable Hamiltonian vector field. Fig. 11 illustrates a local streamline structure that enters the structurally stable Hamiltonian vector field. Herein, symbols used in these drawings and the following description are described. In □ₐ, class-a local streamline structures given in Fig. 11(a) enters. In □_{b+} and □_{b-}, a class-b streamline structure given in Fig. 11(b) enters. In □_{c+} and □_{c-}, an arbitrary number of class-c streamline structures given in Fig. 11(c) may enter. A number appearing in upper right of □ indicates the order of arrangement in the COT representation.

As a simplest flow in an unbounded domain, there is a uniform flow from left to right illustrated in Fig. 10(a). In the drawing, □ₐ and a dotted line portion represent that the class-a streamline structure illustrated in Fig. 11(a) may enter this flow field. The COT representation corresponding to this flow is a_{ϕ}(□ₐₛ). Herein, the notation of □ₐₛ means that the number of structures of □ₐ is s. Specifically, this is transcribed as ${□}_{as} : = {{□}^{1}}_{a} ⋯ {{□}^{s}}_{a} \left(s>0\right) ,$ and ${□}_{as _} : = {λ}_{∼} \left(s=0\right) .$ Note that, it is set in advance that, when there is no streamline structure of □ₐ, a symbol λ_{~} indicating that "there is no structure" is put. Regarding arrangement of the class-a, when the uniform flow flows from left to right, the structures are numbered in order from the bottom. Conversely, in a case where the uniform flow flows from right to left, this is the same as that obtained by interchanging the top and bottom of the drawing, so that they are numbered in order from the top.

Next, when there is no uniform flow, there is a simple rotating flow in the bounded domain. There are two types in Figs. 10(b) and 10(c) depending on a difference between counterclockwise and clockwise rotational directions. The COT representation of this flow is β_{ϕ-}(□_{b+}, {□_{c-s}}) when the flow direction on an outer physical boundary is counterclockwise, and β_{ϕ+}(□_{b-}, {□_{c+s}}) when this is clockwise. As may be understood herein also, note that the flow direction is also correctly classified in this characterization theory. Any one of b₊₊, b₊₋, and β₊ enters □_{b+} appearing in the COT representation, and any one of b₋₋, b-+, and β₋ enters □_{b-}. An arbitrary number of class-c structures in Fig. 11(c) attached to the boundary may enter □_{c±s}. Specifically, this is represented as follows in double-sign in same order: ${□}_{c \pm s} : = {{□}^{1}}_{C \pm } ⋯ {{□}^{s}}_{C \pm } \left(s>0\right) ,$ and ${□}_{c \pm s} : = {λ}_{\pm } \left(s=0\right)$

Herein also, if there particularly is no structure, it is represented by using a symbol of λ_{±} in double-sign in same order. The class-c structures are arranged with numbers assigned in the counterclockwise direction, and there is a (cyclic) voluntariness as to which is selected first. In order to represent this, it is determined in advance to enclose with {} in the COT representation as a rule.

Fig. 11 illustrates all the local streamline phase structures generated by the structurally stable Hamiltonian vector field and symbols accompanied therewith. Signs "+" and "-" are assigned according to the directions (counterclockwise and clockwise) of the generated streamline structure.

Fig. 11(a) illustrates the class-a streamline structures. The COT representation of a structure a₊ is a₊(□_{b+}). This represents that any one of class-b flow local portion structures b₊₊, b₊₋, and β₊ in a counterclockwise (positive) direction is contained in □_{b+}. The COT representation of a structure a₋ is a₋(□_{b-}). This represents that any one of class-b flow local portion structures b₋₋, b₋₊, and β₋ in a clockwise (negative) direction is contained in □_{b-}. In a structure a₂, an arbitrary number of class-c structures are attached above and below a physical boundary. As for a direction, counterclockwise □_{c+} is on an upper side, and clockwise □_{c-} is on a lower side. Therefore, the COT representation thereof is a₂ (□_{c+s}, □_{c-s}).

Fig. 11(b) illustrates the class-b streamline structure. The class-b structure is a structure having the self-connected separatrix. The COT representation thereof may be b_{±±}{□_{b±}, □_{b±}}, b_{±}∓(□_{b±}, □_{b∓}), β_{±}{□_{c±s}} in double-sign in same order. This is similar to the above-description in that a structure of a circle order is enclosed by {} and a structure of a fixed order is enclosed by (), and a symbol of □_{c±s} := λ_{±} is used in a case where there is no structure attached as □_{c±s}.

Fig. 11(c) illustrates the class-c streamline structure. The class-c represents a streamline structure in a domain enclosed by the ∂-saddle separatrix, that is, a structure having an arbitrary number of ∂-saddle separatrices and a large ∂-saddle separatrix enclosing them on the physical boundary. Depending on a rotational direction of the ∂-saddle separatrix, the COT representation thereof is c_{±}(□_{b±}, □_{c}∓ₛ) in double-sign in same order. Herein, there always is the class-b structure in □_{b±}. An arbitrary number of class-c structures are further attached to □_{c±s} in an inner structure. In a case where there is no structure to be attached, this is represented as λ_{±}. As described above, by recursively embedding the local streamline structure that may enter □ from a basic structure, a streamline structure generated by an arbitrary structurally stable Hamiltonian vector field may be formed. Note that a substructure of the local streamline structure represents a microstructure of a finer flow.

A maximum word representation of a flow pattern disclosed in Patent Literature 1 is obtained by assigning a character string on the basis of the basic flow and local streamline structure, but it cannot be said that its representation power is necessarily sufficient. For example, four class-b patterns of b₊₊, b₋₋, b₊₋, and b₋₊ illustrated in Fig. 11(b) are all represented as Bo in the maximum word representation. This has been a factor that the word representation does not have a one-to-one correspondence. Although the regular expression disclosed in Patent Literature 3 distinguishes such structures, information of a local structure that might enter therein is not represented, so that it is not possible to clearly specify the representation of the structure corresponding to β_{±} and c_{±} in advance. This disadvantage makes it difficult to represent as the data structure when configuring the algorithm that realizes the characterization of the streamlined structure. For example, in a structure as illustrated in Fig. 10(b), a local structure corresponding to □_{b+} (including genus element) is necessary, but even when the genus element enters, it is not possible to distinguish whether the center enters or the physical boundary enters here. Therefore, in a case where a point genus element enters, a character of "σ_{±}" (a sign corresponds to a rotational direction of a periodic orbit around this point) is assigned to □_{b±} as a symbol indicating that a "point" enters here, and in a case where no structure enters and only the genus element enters, characters of the COT representation β_{±}{λ_{±}} that means that the c-class structure is not attached at all to a structure of β_{±}, is assigned to □_{b±}. Furthermore, since the presence of the structure corresponding to □_{c-} is not necessarily required, this information of "nothing enters" is not explicitly expressed in a regular expression.

Table 1 illustrates a correspondence relationship among the maximum word representation, the regular expression, and the COT representation corresponding to the streamline phase structure illustrated in Figs. 10 and 11.

| STREAMLINE PHASE STRUCTURE | MAXIMUM WORD | REGULAR EXPRESSION | COT REPRESENTATION |
|---|---|---|---|
| FIG. 10(a) | *I*, *II* | ∘_{0̸} | a_{ø}(□_{αs}) |
| FIG. 10(b) | *O* | +_{0̸} | *β*_{ø}+(□_{*b*+},{□_{*c*-*s*}}) |
| FIG. 10(c) | *O* | -_{0̸} | *β*_{ø-}(□_{*b*-},{□_{*c*+*s*}}) |
| FIG. 11 (a) a₊ | *Λ*₀ | ∘₀,+₀ | a₊(□_{*b*+}) |
| FIG. 11 (a) a₋ | *A*₀ | ∘₀ (-₀) | a₋(□_{*b*-}) |
| FIG. 11(a) a₂ | A₂ | ∘₂ | a₂(□_{*c*+*s*},□_{*c*-*s*}) |
| **FIG.** 11(b) b₊₊ | *B*₀ | +₀(+₀,+₀) | *b*₊₊{□_{*b*+},□_{*b*+}} |
| FIG. 11(b) b₊₋ | *Bo* | +₀(+₀(-₀) ) | b₊₋(□_{*b*+},□_{*b*-}) |
| FIG. 11(b) b₋₋ | *Bo* | -₀(-₀,-₀) | b₋₋{□_{*b*-},□_{*b*-}} |
| FIG. 11(b) b₋₊ | *B*₀ | -₀(-₀(+₀)) | b₋₊(□_{*b*-},□_{*b*+}) |
| FIG. 11(b) *β*₊ | *B*₂, *C* | +₂ | *β*₊{□_{*c*+*s*}} |
| FIG. 11 (b) *β*₋ | *B*₂, *C* | -₂ | *β*₋{□_{*c*-*s*}} |
| FIG. 11 (b) *β*₊ | *B*₂ | +₂ | *β*₊{*c*₊(□_{*b*+},□_{*c*-*s*})·□_{*c*+*s*}} |
| FIG. 11 (b) *β*₋ | *B*₂ | -₂ | *β*₋{*c*₋(□_{*b*-},□_{*c*+*s*})· □_{*c*-*s*}} |
| FIG. 11 (c) *c*₊ | *C* | +₂ | *c*₊(□_{*b*+},□_{*c*-*s*}) |
| FIG. 11 (c) c₋ | *C* | -₂ | *c₋*(□*_{b-}*,□_{*c*+*s*}) |

Herein, □_{c+s} and □_{c-s} represent that □_{c+} and □_{c-} as many as non-negative integer (s ≥ 0) enter, respectively. In this notation, the class-c local streamline structure that might be contained in a substructure of each structure is represented by □_{c±s}, and λ_{±} is assigned to □_{c±s} as a terminal symbol that does not contain any more structure. Such COT representation is a more strict version of the regular expression. This is extremely useful because it is possible to symbolize including the flow direction and the situation of the local streamline structures that may enter the inside. Note that, two local inner structures □_{b±} under b₊₊ and b₋₋represent structures equivalent to each other, so that there is voluntariness in which one is arranged on which side. As noted above, there also is voluntariness in how to determine an identification number of the c-class local structure in β_{±}, that is, how to arrange them, so that in a case where there is such voluntariness, each structure is enclosed by curly brackets {}. On the other hand, the local structure in which the order is naturally determined is enclosed by parentheses (). By adopting this COT representation, it is possible to accurately represent the streamline phase structure as the data structure after eliminating ambiguity. Note that the self-connected separatrix and ∂-saddle separatrix in the conventional study of the incompressible vector field are replaced with homoclinic saddle connection and ∂-saddle connection, respectively.

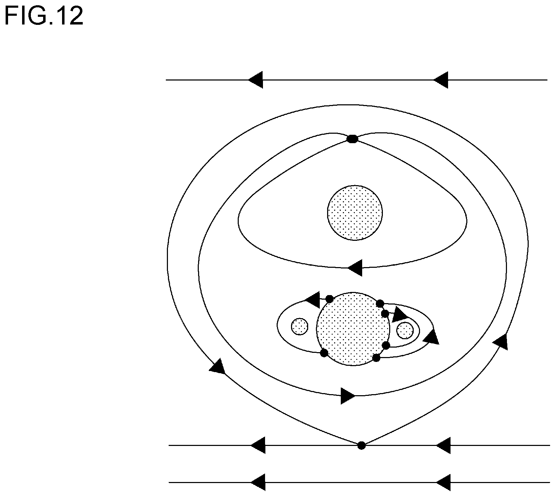

As an example, by using this COT representation, a word representation is given to a flow pattern illustrated in a flowchart in Fig. 12. First, in this flow, there is a local substructure of a₊ in the uniform flow in the unbounded domain. As illustrated in Fig. 11(a), an a₊(□_{b+}) structure must necessarily include the class-b structure as the substructure. Actually, looking at Fig. 12, it may be seen that there is a b₊₋ (□_{b+}, □_{b-}) structure in □_{b+}. Therefore, the structure up to this point is represented as a_{ϕ}(a₊(b₊₋(□_{b+}, □_{b-}))) in the COT representation. Furthermore, when looking at an inner structure of b₊₋, since there is the genus element of the physical boundary in the counterclockwise direction in □_{b-}, □_{b-} := β₋{λ₋} representing the physical boundary with clockwise periodic orbits around the same is put therein. There is a β₊{□_{c+s}} structure in □_{b+} and two c-classes c+ are attached thereto; however, there is one physical boundary accompanied with the periodic orbit in c+ on a left side and this reaches a terminal, so that the symbol thereof is c₊(β₊{λ₊}, λ₋) . In c+ on a right side, a c-structure further enters a lower portion. Therefore, as a □_{b+} structure in the COT representation c₊(□_{b+}, □_{c-s}) of c+, σ₊ that means a point accompanied with the counterclockwise periodic orbit enters, and c₋(β₋{λ₋}, λ₊) enters □_{c-s}. Therefore, in β₊{□_{c±s}}, □_{c+s} = c₊(β₊{λ₊}, λ₋) ·c₊(σ₊, c₋(β₋{λ₋}, λ₊)) may enter.

When summarizing the above, the COT representation of the flow pattern in Fig. 12 may be given as a_{ϕ}(a₊(b₊₋(β₊{c₊(β₊{λ₊} , λ₋)·c₊(σ₊, c₋(β₋{λ₋}, λ₊))}, β₋{λ₋}))). Herein, the symbol λ_{±} indicating that "no structure is contained" is important as a program, but is redundant when represented as the character. Therefore, this λ_{±} may be omitted, and it is possible to represent without confusion even if this is abbreviated as a_{ϕ}(a₊(b₊₋(β₊{c₊(β₊) ·c₊(σ₊, c-(β₋))}, β₋))). The present inventors find that there is the algorithm for converting this COT representation into the regular expression, and further, the regular expression may be converted into the maximum word representation, so that all the representations are automatically obtained from the COT representation. Actually, a regular expression O_{ϕ}(O₂(+₀(+₀(+₀, +₀(+₀) , -₀)))) and a maximum word representation IA₀B₀B₂CC may be obtained from the COT representation of the flow pattern in Fig. 12.

On the basis of the above-described theory, hereinafter, all streamline structures that may be taken in a topological manner are classified and the characters (COT representation) are assigned thereto.

### Two-Dimensional Structure

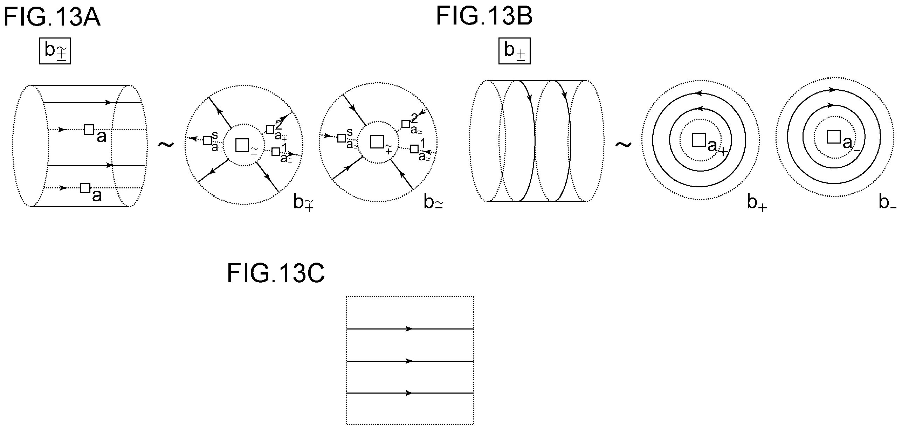

Theorem 1 shows that three types of flows enter the two-dimensional domains divided by the set Bd(v) of border orbits in the ss-saddle connection diagram Dₛₛ(v). Herein, this two-dimensional domain structure is first defined, and then the characters (COT representation) corresponding thereto are given. The open rectangular domain illustrated in Fig. 9(a) includes a non-closed orbit group in the vicinity of the ss-separatrix connecting the source structure and the sink structure, but this conversion algorithm does not assign a symbol thereto. That is, this is a default structure. The characters (COT representation) are assigned to two two-dimensional structures other than this. They form class-b± and class-b_{~±} elements as illustrated in Table 3. Fig. 13 illustrates a structure (two-dimensional structure) representing the orbit group of (Bd(v))^{c} given by theorem 1.

### Two-Dimensional Structure: b_{~±}

The structure of the open annular domain illustrated in Fig. 9(b) is in a situation filled with the non-closed orbits from the outside to the inside. For this, a symbol b_{~±} is assigned as in Fig. 13 (a). For a sign _{~±} attached to the symbol, when the orbit group flows from the outer boundary to the inner boundary of an annulus, this is set to ~- for representing that the flow is sucked into the center. On the other hand, in a case where the orbit group spreads from the inner boundary to the outer boundary, this is set to ~+. The sink/source structure always enters the inner boundary, and such set of Bd(v) structures is denoted by □_{~±}. Since an arbitrary number (s ≥ 0) of class-a_{~±} orbit structures of Bd(v) denoted by □_{a~±} may be put in each of the non-closed orbits filling the domain, a symbol representing the same is set to □_{a~±s}. An arrangement of structures corresponding to □_{a~±s} is not uniquely determined in a circular order. On the basis of the above-described consideration, the COT representation is b_{~±}(□_{~±}, {□_{a~±s}}) in double-sign in same order. Note that, as for definitions of class-~± and class-a~± structure groups that may enter □_{~±} and □_{a~±s}, respectively, please refer to Table 3. As in the notation in a case of □ₐₛ of the incompressible flow, □_{a~±s} is set to ${□}_{a ∼ \pm s} : = {{□}^{1}}_{a ∼ \pm } ⋯ {{□}^{s}}_{a ∼ \pm } \left(s>0\right) ,$ and ${□}_{a ∼ \pm s} : = {λ}_{∼} \left(s=0\right) ,$ and a symbol λ_{~} is used as is the case with the structure stable Hamiltonian vector field for representing that "nothing is contained".

### Two-Dimensional Structure: b_{±}

Fig. 9(c) illustrates a situation in which an open disk domain is filled with the periodic orbits. The streamline structure corresponding to this is a structure b_{±} illustrated in Fig. 13(b). A sign + is assigned when the periodic orbits rotate counterclockwise (in a positive direction) and a sign - is assigned when they rotate clockwise (in a negative direction). The structure in the same is also the element of Bd(v), but since it is a class-α orbit structure that enters there, this is denoted by □_{*α*±}, and its definition is given in Table 3. Unlike the non-closed orbit, the structure such as □ₐ does not enter the periodic orbit, so that this COT representation is given as b_{±}(□_{α±}) in double-sign in same order.

Hereinafter, the structure of the orbit group that might be included in the domain divided by Bd(v) is selected from above. Herein, for use in the COT representation defined for Bd(v), sets defined from the structure of (Bd(v))^{c} of □_{bϕ}, □_{b+}, □_{b-}, □_{b~+}, and □_{b~-} are defined as follows. ${□}_{b +} = \left\{{b}_{∼ \pm }, {b}_{+}\right\}$ ${□}_{b -} = \left\{{b}_{∼ \pm }, {b}_{-}\right\}$ ${□}_{b ∼ +} = \left\{{b}_{∼ +}\right\}$ ${□}_{b ∼ -} = \left\{{b}_{∼ -}\right\}$

### Basic Structure

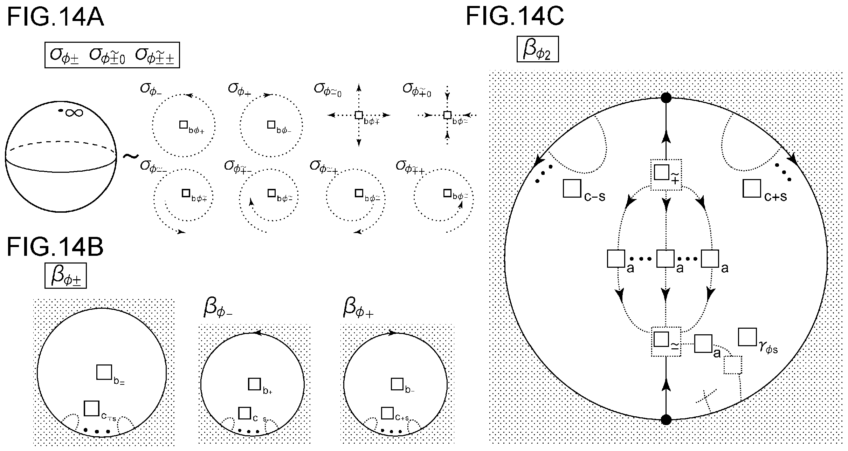

Topologically, the plane may be identified with the spherical surface S from which the point of infinity is removed. The following basic flows are present on the spherical surface S. Hereinafter, these fluid structures are referred to as a "basic structure" or a "root structure". Fig. 14 illustrates the basic structure on the spherical surface S.

### Basic Structure: σ_{ϕ±}, σ_{ϕ~±0}, σ_{ϕ~±±} and σ_{ϕ~±}∓

The flow field when there is no physical boundary at all in the plane may be identified with the flow on the spherical surface as illustrated in Fig. 14(a). This flow of finite type on the spherical surface gives the flow in the annular domain except for two zero-dimensional point structures on both poles. At that time, the COT representation of this structure needs to be classified depending on the orbit structure contained therein. When this annular domain is filled with an orbit group structure b_{±} formed of the periodic orbits given by the structure b_{±}, a representation σ_{ϕ}∓(□_{bϕ±}) is assigned, wherein □_{bϕ±} = b_{±} (□_{*α*±}) . On the other hand, when the annular domain is filled with a class-~± non-closed orbit group structure b_{~±} of source/sink, the COT representation thereof is any one of σ_{ϕ~}∓₀(□_{bϕ~±}) , σ_{ϕ~}∓_{±}(□_{bϕ~±}) , and σ_{ϕ~}∓∓(□_{bϕ~±}) depending on the rotational direction of the orbit around the source/sink. That is, around the source/sink that is a point at the point of infinity, when the non-closed orbit group does not rotate, σ_{ϕ~}∓₀(□_{bϕ~±}) in double-sign in same order, when this rotates counterclockwise, σ_{ϕ~}∓₊(□_{bϕ~±}) in double-sign in same order, and when this rotates clockwise, σ_{ϕ~}∓₋(□_{bϕ~±}) in double-sign in same order. Herein, □_{bϕ~±} = b_{~±} (□_{~±}, {□ₐₛ}). Note that the signs assigned to the symbols of σ_{ϕ~±0}, σ_{ϕ~}∓∓, and σ_{ϕ~}∓_{±} are opposite to the signs of the COT representation of a two-dimensional orbit group structure contained therein because the sign is assigned to the structure of the flow around the point corresponding to the point at infinity. For example, for embedding the periodic orbit in the counterclockwise direction (that is, + direction) with the representation of b+ inside, a flow in a clockwise direction (that is, - direction) must occur around the point at the point of infinity. Therefore, specific COT representation is ${σ}_{φ -} \left({□}_{b φ +}\right) ,$ ${□}_{b φ +} = {b}_{+} \left({□}_{α +}\right) .$

### Basic Structure: β_{ϕ±} and β_{ϕ2}

Suppose that the spherical surface includes some physical boundaries. At that time, it is possible to select one of them as a special boundary, and introduce spherical polar coordinates such that a north pole is included in the boundary. At that time, the flow on the spherical surface may be identified with an inner flow in the two-dimensional bounded domain through a stereographic projection associated with this coordinate system. Fig. 14(b) illustrates a flow that is a child of the root and includes no source/sink structure at all on an outer physical boundary. The COT representation is given as β_{ϕ-} (□_{b+}, {□_{c-s}}) when the flow on the outer boundary is counterclockwise. Note that this flow is the same as that of the basic structure of the structurally stable Hamiltonian vector field illustrated in Fig. 10(b). Although this structure must constantly include a class-b+ structure in □_{b+}, it is possible to attach an arbitrary number of class-c- orbit structures on the outer boundary. When the flow direction on the outer boundary is clockwise, all the signs are inverted to obtain the basic structure having the COT representation of β_{ϕ+}(□_{b-}, {□_{c+s}}). Note that, in both cases, □_{c±s} that means (s ≥ 0) class-c orbit structures may be specifically represented as follows in double-sign in same order.

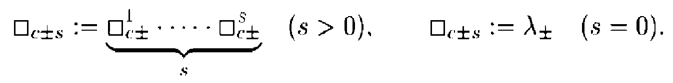

A basic flow structure is illustrated in which there is at least one source/sink structure connected to the outer physical boundary illustrated in Fig. 14(c). A basic structure β_{ϕ2} is the basic flow structure illustrated in Fig. 14(c). A pair on a leftmost side is selected from the source/sink structures as a class-~± special orbit structure, and a class-γ_{ϕ} orbit structure (Table 3) is assigned to other source/sink structures. In addition to this, an arbitrary number of class-c_{±} orbit structures may be attached to right and left along the boundary. This situation is represented in the COT representation by □_{~±} for the special pair of source-sink and by □_{γϕs} for an arbitrary number of class-γ_{ϕs} orbit structures. Specifically, this may be represented as follows.

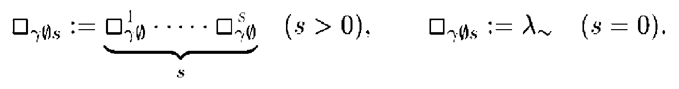

Since there is the special pair of source-sink, an arbitrary number of class-a orbit structures connecting them may be attached. They are represented as □ₐₛ in the COT representation. Note that, refer to Table 3 for the definition of the class-a structure group that may enter □ₐₛ. As in a manner similar to the notation in a case of the incompressible flow, □ₐₛ is determined as: ${□}_{as} : = {{□}^{1}}_{a} ⋯ {{□}^{s}}_{a} \left(s>0\right) ,$ and ${□}_{as} : = {λ}_{∼} \left(s=0\right) .$ In summary, the COT representation of this basic structure is given as β_{ϕ2}({□_{c+s}, □_{~+}, □_{c-s}, □_{~-}, □_{γϕs}}, □ₐₛ) by arranging each structure attached to the outer boundary counterclockwise in a circular order.

### Zero-Dimensional Point Structure and One-Dimensional Structure of Flow of Bd(v)

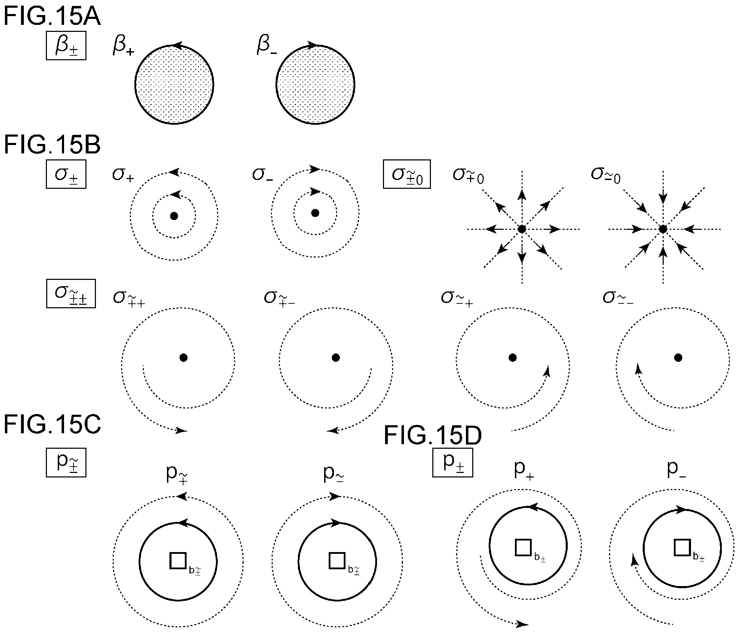

Next, classification of the zero-dimensional point structure and the one-dimensional structure of Bd(v) forming the ss-saddle connection diagram Dₛₛ(v) defined by the flow of finite type v on the curved surface S, and the COT representation corresponding to the same are given. According to the above-described theory, since it is represented as Bd(v) = Sing(v) U ∂Per(v) U ∂P(v) U Pₛₑₚ (v) U ∂ₚₑᵣ(v), the zero-dimensional point structure and the one-dimensional structure that realize Bd(v) are introduced corresponding to each set. Note that a set in which each one-dimensional structure enter might change depending on orbit group information around the same. Fig. 15 illustrates an example of the zero-dimensional point structure and the one-dimensional structure of Bd(v).

### Structures of ∂ₚₑᵣ(v), and Sing(v)

### One-Dimensional Structure: β_{±}

By definition, the flow of ∂ₚₑᵣ(v) refers to the periodic orbit flowing along the physical boundary. In the structurally stable Hamiltonian vector field, a symbol β is given to the physical boundary as illustrated in Fig. 11(b). Taking consistency with this, it is represented by the same symbol that a class-c structure enclosed by the ∂-saddle separatrix is not attached at all to the physical boundary. That is, the COT representation is given as β₊{λ₊} when the flow on the physical boundary is counterclockwise, and as β₋{λ₋} when the flow is clockwise (refer to Fig. 15(a)). These physical boundaries enter class-~± and class-α_{±} structures.

### Zero-Dimensional Point Structure: σ_{±}, σ_{∼±0}, σ_{~±±}, and σ_{~±}∓

An element of Sing(v)\Dₛₛ(v) is the zero-dimensional point structure (isolated structure). The point structures may be classified by the orbit around the same. When the point is the center accompanied by the counterclockwise or clockwise periodic orbit around the same, the COT representation thereof is given by σ₊ and σ₋, respectively. On the other hand, when the point is the source or the sink, the COT representation is given by σ_{∼±0}, σ_{~±±}, and σ_{~±}∓ along with the rotational direction of the orbit around the same (refer to Table 2 and Fig. 15(b)). These point structures enter the class-~± structure.

| | COUNTERCLOCKWISE | CLOCKWISE | NO ROTATION |
|---|---|---|---|
| SOURCE | σ_{~++} | σ_{~+-} | σ_{~+0} |
| SINK | σ_{~-+} | σ_{~--} | σ_{~-0} |

### Structures Entering ∂P(v) and ∂Per(v)

### One-Dimensional Structure: p_{~±}, p_{±}

The sets ∂P(v) and ∂Per(v) are the one-dimensional structures defined as boundary sets of non-closed orbits and periodic orbits, respectively. The limit cycle is the periodic orbit in which either an inner side or an outer side thereof is a limit orbit of the non-closed orbit. Since this is not an element of intP(v), this is a structure that cannot be an element of a set Pₛₑₚ(v). At that time, classification is required depending on structures on the outer side and the inner side of the limit cycle. That is, one is a structure of the periodic orbit in an outer domain of the limit cycle illustrated in Fig. 15(c), and the other is a structure in which the limit cycle illustrated in Fig. 15(c) is the ω(α)-limit set of the non-closed orbits in the outer domain. The former structure is denoted by p_{~±}. In order for this periodic orbit to be the limit cycle at that time, this must be the limit orbit of the non-closed orbit from the inside (that is, the border orbit). Therefore, the two-dimensional structure of b_{~±} is put therein. Therefore, the COT representation thereof is p_{~±}(□_{b~±}). The latter structure is represented by a symbol p_{±}. At that time, since this is a limit periodic orbit from the outside, the structure of an inner two-dimensional limit orbit group may be any structure. Therefore, the COT representation thereof may be p_{±}(□_{b±}) in double-sign in same order.

### Structures Entering ∂P(v), ∂Per(v), and Pₛₑₚ(v)

The one-dimensional structure that might enter any of structure sets of ∂P(v), ∂Per(v), and Pₛₑₚ(v) has a non-closed orbit including the structure of the saddle separatrix or ss-separatrix illustrated in Fig. 8. As the two dimensional structures entering inner and outer portions thereof, a case of the domain where one is filled with the non-closed orbits and the other is filled with the periodic orbits (at that time, ∂P(v) or ∂Per(v)), and a case of the domain where both the sides are filled with the non-closed orbits (at that time, the element of Pₛₑₚ(v)) must be considered.

First, since there are four separatrices connected to the saddle, there are three following possibilities in consideration of local flow directions of the separatrices.

(S1) One is connected to the source structure, one is connected to the sink structure, and remaining two are self-connected saddle separatrices.

(S2) There are two self-connected saddle connections.

(S3) The two are connected to the source (sink) structure. Note that the remaining two cannot be the self-connected separatrices from the flow direction.

Among them, a case of (S3) cannot has the non-closed orbit illustrated in Fig. 8, so that it is only required to consider (S1) and (S2). On the other hand, there are three separatrices at the ∂-saddle, but since two of them need to run on the boundary, there is only one degree of freedom. Therefore, there are following two possibilities of the connected structure.

(S4) This has the ∂-saddle separatrix connected to another ∂-saddle on the same boundary.

(S5) This is connected to the source/sink structure inside the domain.

In a case of (S4), there is no problem because the non-closed orbit is obviously formed, but a case of (S5) depends on a surrounding situation. That is, in this case, the non-closed orbit does not occur alone. However, in relation to an Euler number, it is required to recognize the presence of one ∂-saddle and at least one different ∂-saddle. Therefore, when the ∂-saddle has the ∂-saddle separatrix, an entire structure might include the non-closed orbit. From above, four structures corresponding to (S1), (S2), (S4), and (S5) are hereinafter considered.

### One-Dimensional Structure: a_{±} and q_{±}

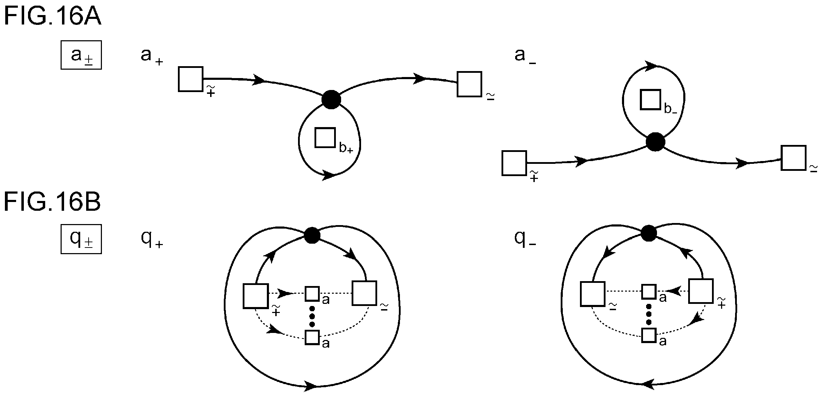

Fig. 16 illustrates (S1)-series one-dimensional structures. These structures are classified according to a positional relationship among the source structure, sink structure, and self-connected saddle separatrix.

First, a structure illustrated in Fig. 16(a), that is, the structure in which the source/sink structure is present outside an enclosing domain of the self-connected saddle separatrix is denoted by a_{±}. A sign is determined to be a+ and a₋ in a case where there is the self-connected saddle separatrix on a lower side and on an upper side, respectively, with respect to a flow from left to right. In this structure, the periodic orbit or non-closed orbit might enter the self-connected saddle separatrix. In a case where the periodic orbit enters among them, the rotational direction thereof is automatically determined, so that a structure set defined by □_{b+} enters a₊, and a structure set defined by □_{b-} enters a₋. Note that, since this is the same structure as the class-a orbit structure (Fig. 11(a)) appearing in the structurally stable Hamiltonian vector field, the same COT representation is assigned.

Next, a structure illustrated in Fig. 16(b), that is, the structure in which the source/sink structures are present inside the enclosing domain of the self-connected saddle separatrix is denoted by q_{±}. A sign is q₊ when the direction of the outer self-connected saddle separatrix is counterclockwise, and q- when the direction is clockwise. In this structure, there may be the enclosed source/sink structures and an arbitrary number (s ≥ 0) of elements of the structure set of □ₐₛ connecting them, so that the COT representation thereof is q_{±} (□_{∼+}, □_{∼-}, □ₐₛ).

### One-Dimensional Structure: b_{±±} and b_{±}∓

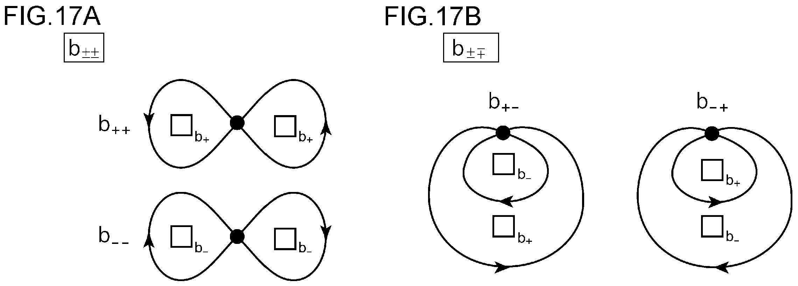

Fig. 17 illustrates (S2)-series one-dimensional structures. This is the same structure as that used in the classification of the structurally stable Hamiltonian vector field, that is the structure illustrated in Fig. 11(b). Therefore, the same COT representation is given. That is, the structures are classified according to a positional relationship between the two self-connected saddle separatrices.

First, a structure illustrated in Fig. 17(a), that is, the structure in which enclosing domains of the two self-connected saddle separatrices are on the outer sides of each other is denoted by b_{±±} in double-sign in same order. A sign is b++ when the rotational direction of the two self-connected saddle separatrices is counterclockwise, and b₋₋when the direction is clockwise. The two-dimensional structures might enter inner domains enclosed by the two self-connected saddle separatrices. In a case where the periodic orbits enter, the rotational direction thereof is automatically determined, and there is a degree of freedom in an arrangement order of the two domains, so that they are enclosed by {}, and the COT representations thereof are b₊₊{□_{b+}, □_{b+}} and b₋₋{□_{b-}, □_{b-}}.

Next, a structure illustrated in Fig. 17(b), that is, the structure in which, in an enclosing domain of one self-connected saddle separatrix, the other self-connected saddle separatrix is included is denoted by b_{±}∓ in double-sign in same in order. A sign is b+- when the direction of the outer self-connected saddle separatrix is counterclockwise, and b-+ when the direction is clockwise. From this method of determining the sign, in a case where the orbit group inside is the periodic orbit, it is automatically determined that the rotational directions are opposite to each other. Therefore, the COT representations thereof are b₊₋(□_{b+}, □_{b-}) and b₋₊(□_{b-}, □_{b+}). Herein, it is determined to order the arrangement of the inner structures so as to match signs under b.

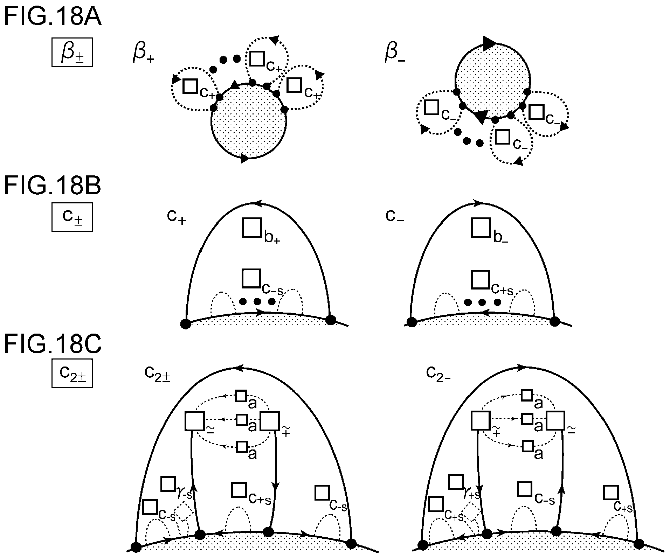

Fig. 18 illustrates (S4)-series one-dimensional structures.

### One-Dimensional Structure: β_{±}

A structure illustrated in Fig. 18(a) corresponds to the physical boundary to which an arbitrary number of α-saddle separatrices are attached. If no α-saddle separatrix is attached, a form illustrated in Fig. 15(a) is obtained, and the COT representation thereof is β_{±}{λ_{±}}. On the other hand, when one or more α-saddle separatrices are attached to the boundary, the structure is the same as β_{±} in Fig. 11(b) given in the structurally stable Hamiltonian vector field. Therefore, as the COT representation at that time, β₊{□_{c+s}} is given in a case where the flow is counterclockwise along the boundary, and β₋{□_{c-s}} is given in a case where the flow is clockwise (refer to Fig. 18(a)). That is, the following symbols obtained by arranging each structure counterclockwise in a circular order enter □_{c±s}. ${□}_{c \pm s} : = {{□}^{1}}_{c \pm } ⋯ {{□}^{s}}_{c \pm } \left(s>0\right)$ ${□}_{c \pm s} : = {λ}_{\pm } ⋯ \left(s=0\right)$

### One-Dimensional Structure: c_{±} and c_{2±}

One-dimensional structures c_{±} and c_{2±} correspond to the (S4)-series. As illustrated in Figs. 18(b) and (c), they may be classified according to a structure entering an inner portion enclosed by the α-saddle separatrix.

First, when the two-dimensional structure filled with the periodic orbits or non-closed orbits enters, that is, when the source/sink structures connected to the α-saddle do not enter, a structure illustrated in Fig. 18(b) is obtained. This is denoted by c_{±}. A sign is + when a rotational direction is counterclockwise, and - when the direction is clockwise in an orbit along the α-saddle separatrix and the boundary. At that time, when the inner orbit group is formed of the periodic orbits, the direction thereof is automatically determined. Note that an arbitrary number of c_{±} structures may be further included therein, but the rotational direction of the inner portion is opposite in this case. On the basis of this, a set of c_{±} structures is defined as: ${□}_{c +} = \left\{C+\right\} ,$and □_{c-} = {c₋}. When the structure set of □_{c±s} is defined in double-sign same order in the sense that arbitrary number (s ≥ 0) of them are arranged, the COT representation thereof is c_{±}(□_{b±}, □_{c∓s}) in double-sign same order.

Next, in a case where the source/sink structures enter the inner portion, these structures need to be connected to the α-saddle on the same boundary in relation to the Euler number. That is, the structure illustrated in Fig. 18(c), that is, the structure in which the slidable α-saddle is enclosed by the α-saddle separatrix is obtained. This is denoted by C_{2±}. In general, any number of slidable α-saddles may be attached to the boundary, so that a rightmost one is selected, and a pair of source/sink structures corresponding to the same is represented as □_{∼±}. The structure set □ₐₛ indicating that there may be an arbitrary number (s ≥ 0) of structure sets □ₐ connecting the source/sink structures is included. Furthermore, on the boundary, an arbitrary number (s ≥ 0) of c_{±} structures may be present depending on their directions, and in addition to this, a set □_{γ∓s} representing an arbitrary number of (s ≥ 0) of slidable α-saddle structures may be present. Note that the structure of □_{γ±s} is always on the left side because the rightmost one of a total of (s + 1) slidable α-saddle structures as a whole is selected and represented as □_{~±}. Herein, in order to give the COT representation of this structure, one rule is determined for the arrangement of the inner structures. That is, the rule is such that "in a case where there is a structure enclosed by the α-saddle separatrix on a circle boundary inside, the inner structures are arranged in a counterclockwise direction as seen from the inside of the boundary. On the other hand, in a case of attaching to an outer boundary of β_{ϕ2} that has just appeared, the structures are arranged in a clockwise direction as seen from the inside of the boundary (conversely, in the counterclockwise direction as seen from a portion where a fluid is present)". According to this rule, the COT representation of the structure illustrated in Fig. 18(b) may be obtained by arranging the structures from a rightmost side as c_{2±}(□_{c∓s}, □_{~±}, □_{c±s}, □_{-∓}, □_{γ∓s}, □_{c∓s}, □ₐₛ) considering that the structure is attached to the inner boundary. Note that, when the rules are determined in this manner, the structure is such that the structures in the clockwise direction are always arranged regardless of the inner and outer boundaries.

### One-Dimensional Structure: a₂, γ_{ϕ∼±}, and γ_{∼±±}

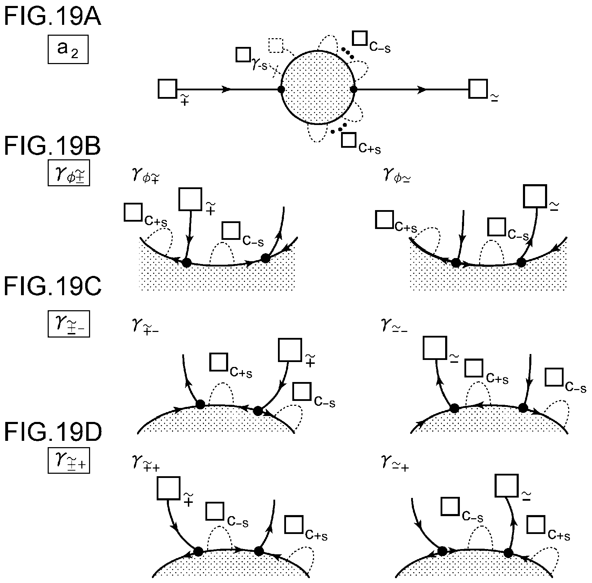

Fig. 19 illustrates (S5)-series one-dimensional structures. The one-dimensional structures basically correspond to the slidable α-saddle connecting a pair of source/sink structures on the boundary. For convenience of a later algorithm configuration, in a case where there is a plurality of such structures, one of them is treated as a special structure.

The structure illustrated in Fig. 19(a) is the structure representing a special one of the slidable saddles connecting the pair of source/sink structures, and is denoted by a₂. Any number (s ≥ 0) of structures □_{c±} enclosed by the α-saddle separatrix may be attached to the physical boundary depending on a flow direction on the boundary. This is denoted by □_{c±s}. Any number of other slidable α-saddle structures may be attached, but when a lowermost slidable α-saddle is especially selected, s ≥ 0 structures □_{γ-} of other slidable α-saddle are present only on an upper side thereof.

This is represented as □_{γ-s}. Finally, the COT representation may be made a₂(□_{c+s}, □_{c-s}, □_{γ-s}) by arranging the structures counterclockwise on the boundary according to a rule of arrangement of the structures of the COT representation with respect to the structure attached to an inner circle boundary. Note that □_{γ-s} enters only a left side of □_{c-s} by definition of a structure γ_{∼±±} to be introduced later.

After selecting the special slidable α-saddle connecting the source/sink structures, all other slidable α-saddles need to be treated equally. Structures added here must be classified by the structure of the boundary to which they are attached. First, an arbitrary number of structures may be attached to an outer circle boundary of β_{ϕ2}, but in the definition of β_{φ2}, a symbol of □_{~±} is assigned to a pair structure of the source-sink that is on the leftmost side always, so that all the others are on the right side. Since the flow proceeds from top to bottom along a right boundary, s ≥ 0 slidable α-saddlesmay also be added in the same direction. At that time, two α-saddlesare added in relation to the Euler number. This makes it possible to sandwich the structure of □_{c±s} therebetween. With reference to Fig. 14(d), the structure of □_{c+s} enters the right boundary along the flow. Therefore, in order to add the structure of slidable α-saddle beyond this, a structure as illustrated in Fig. 19(b) is required. This structure is denoted by γ_{ϕ∼±}. The sign is γ_{ϕ∼+} when a newly added structure is the source structure, and γ_{ϕ∼-} when this is the sink structure. The COT representations of the respective structures are γ_{ϕ∼+} (□_{c+s}, □_{~+}, □_{c-s}) and γ_{ϕ∼-} (□_{c+s}, □_{c-s}, □_{∼-}) because the structure is read counterclockwise (clockwise as seen from the outside inside) from left to right in a case of the structure attached to the outer boundary. Note that the existing sink (source) structure is connected to the α-saddle different from the α-saddle connected to the added source (sink) structure.

Finally, the structure of the slidable α-saddle attached to the boundary at a₂ or c₂ includes two types of a structure added from a downstream side of the flow along the boundary (Fig. 19(c)) and a structure added from an upstream side (Fig. 19(d)) . They are denoted by γ_{∼±-} and γ_{-±+}, respectively. Then, the sign is determined depending on whether a newly added structure is the source structure or sink structure. The corresponding COT representations are γ_{∼±-}(□_{c-s}, □_{~+}, □_{c+s}) , γ_{∼--}(□_{c-s}, □_{c+s}, □_{∼-}), γ_{~++}(□_{c+s}, □_{c-s}, □_{~+}), and γ_{∼-+}(□_{c+s}, □_{∼-}, □_{c-s}) because the structure is read counterclockwise on the inner boundary. Note that there are s ≥ 0 structures of γ_{~±+}, and s ≥ 0 structures of γ_{∼±-} in herein introduced □_{γ+s} and □_{γ-s}, respectively.

### Structure Entering Pₛₑₚ(v)

### One-Dimensional Structure: a_{~±} and q_{~±}

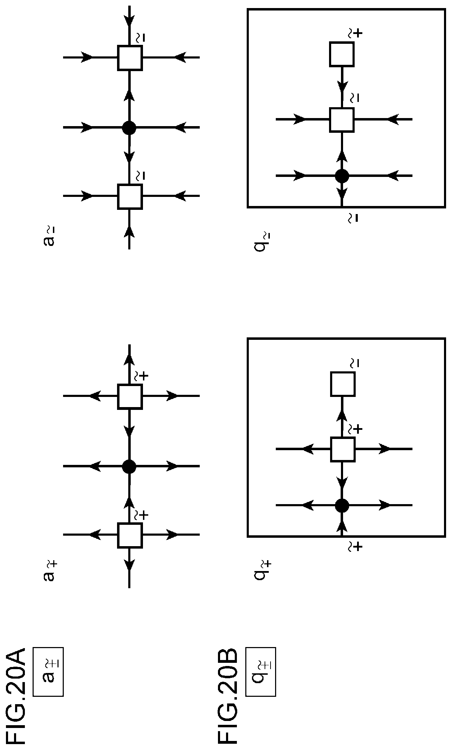

Fig. 20 illustrates (S3)-series one-dimensional structures. They become the slidable saddles since two same source/sink structures are attached to the saddle. The structures are classified according to a positional relationship of the ss-component connected to the slidable saddle. At that time, since all the neighborhood orbits are two-dimensional domains (open rectangular domains) filled with the non-closed orbits, they always are elements of Pₛₑₚ(v). A structure corresponding to the slidable saddle in Fig. 20(a) present outside the ss-component to which the slidable saddle is connected is denoted by a_{~±}. A structure in Fig. 20(b) present inside the ss-component to which the slidable saddle is connected corresponds to the slidable saddle. This is denoted by q_{~±}. A sign is a_{~+} in a case where a source structure □_{∼+} is attached on both sides, and a_{∼-} in a case where a sink structure □_{∼-} is attached on both sides. The order of these source/sink structures may be freely selected, so that the COT representations are a_{~±}{□_{~±}, □_{~±}} and q_{~±}(□_{~±}) in double-sign in same order.

As described above, all the streamline structures (that is, the streamline structures forming an arbitrary flow pattern in the two-dimensional domain) generated by the flow of finite type on the curved surface S and the characters (COT representation) corresponding thereto are given. Table 2 illustrates a correspondence relationship between each streamline structure and the characters (COT representation). A list of sets of structures included in each COT representation is illustrated in Table 3.

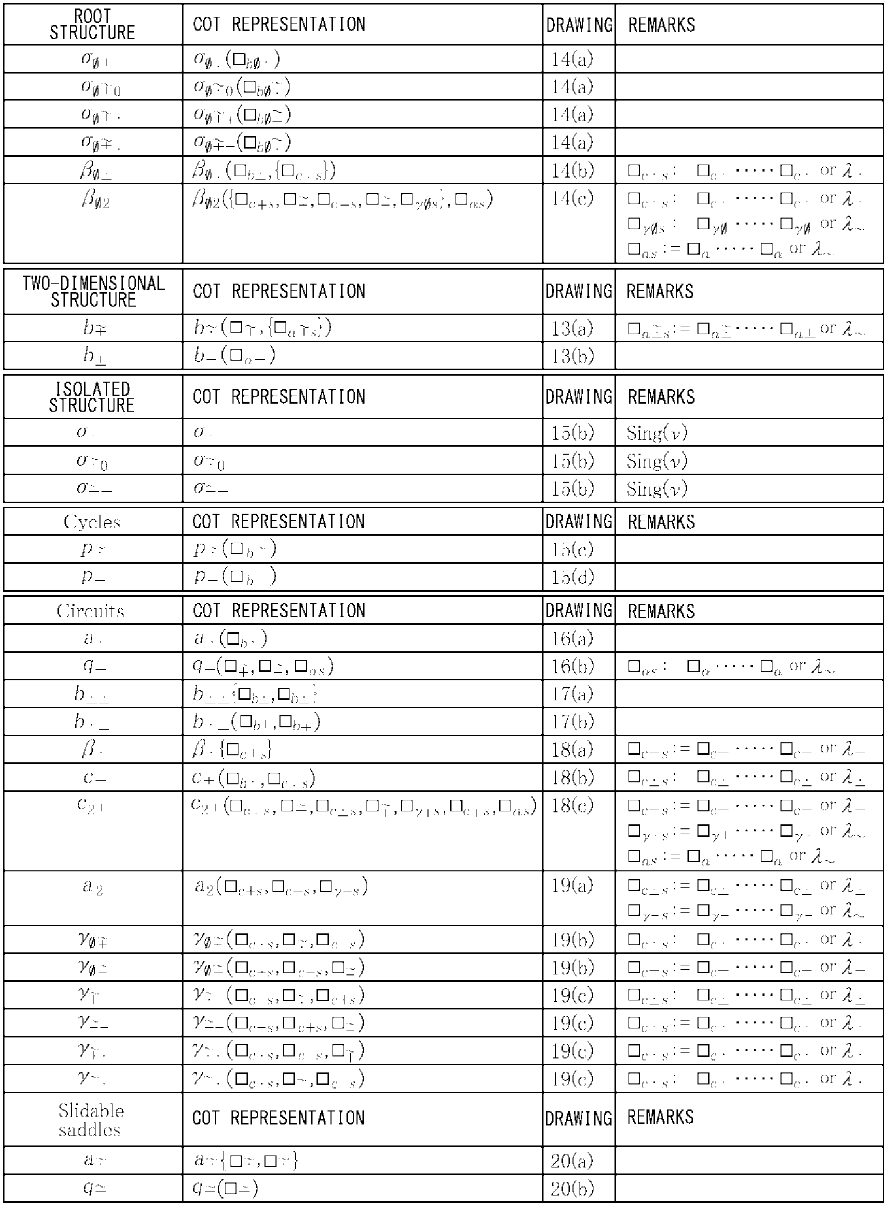

| STRUC TURE | FORMING STRUCTURE GROUP | REMARKS | |
|---|---|---|---|
| class-b_{0̸±} | □_{b0̸±} | {*b*_{±}} | used in σ_{0̸±} |
| class-b_{0̸±̃} | □_{b0̸±̃} | {*b*_{±̃}} | used in σ_{0̸±̃} |
| class-b₊ | □_{b+} | {*b*_{±̃·}*b*₊} | 2D orbit structures |
| class-*b*₋ | □*b*₋ | {*b*_{±̃·}*b*₋} | 2D orbit structures |
| class-b_{±̃} | □_{b±̃} | {*b*_{±̃}} | 2D orbit structures |
| class-+̃ | □_{±̃} | {*p*₌,*b*_{±±},*b*_{±}-,*q*_{±},*β*_{±},*a*_{+̃},*σ*_{+̃0},*σ*_{+̃=}} | source structures |
| class--̃ | □_{-̃} | {*p*₋,*b*₊₊,*b*₊-,*q*₊,*β*₊,*a*≃,*σ*≃₀,*σ*≃₌} | sink structures |
| claas-*a*₊ | □₊ | {*p*_{+̃},*b*₊₊,*b*₊₋,*q*₊,*β*₊,*σ*₊} | used in *b*₊ |
| class-*a*₋ | □_{*a*-} | {*p*≃,*b*₋₊,*b*₋₋,*q*₋,*β*₋,*σ*₋} | used in *b*₋ |
| class-a | □*ₐ* | {*a*_{±},*a*₂} | |
| class-*a*≃ | □*ₐ*≃ | {*q*_{+̃,}*a*_{±,}*a*₂} | |
| class-a_{+̃} | □_{*a*+̃} | {*q_{-,}a*_{±,}*a*₂} | |
| class-c₊ | □_{*c*-} | {*c*₊,*c*₂₊} | |
| class-*c*₋ | □_{*c*-} | {*c*₋,*c*₂₋} | |
| classy-γ_{0̸} | □*_{γ0}* | {γ_{0̸±}} | |
| class-γ₊ | □_{*γ*+} | {γ_{±̃+}} | |
| class-*γ*₋ | □*_{γ}* | {γ_{±̃-}} | |

### Tree Representation

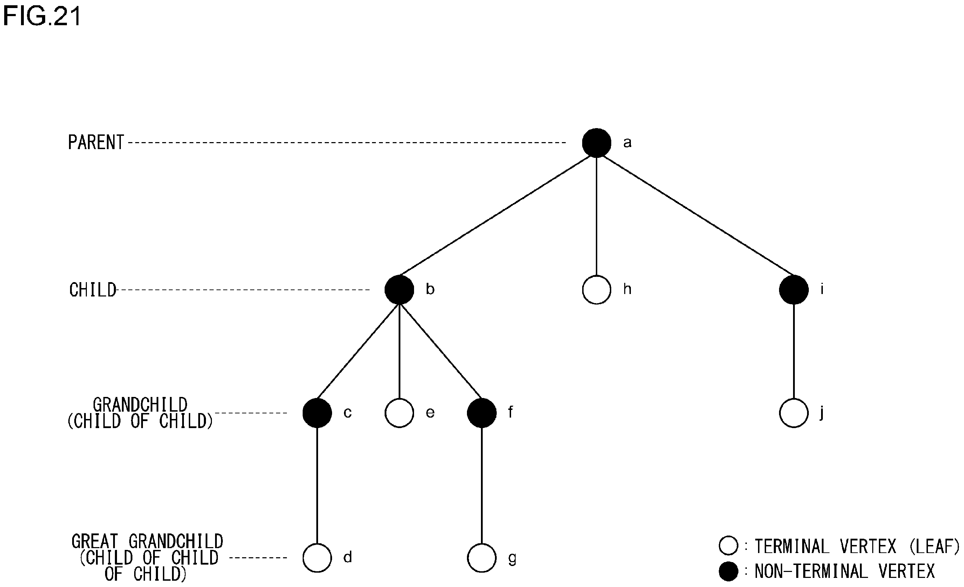

Next, basic matters regarding a "tree representation" used in this specification are described with reference to Fig. 21. Fig. 21 illustrates an example of a general tree representation. As illustrated, the tree representation is a graph having a structure in which vertices are connected by lines. The vertices of the tree are roughly divided into two types: ones located at a terminal end of the tree (o) and others (•). The former (d, e, g, h, and j) is referred to as a terminal vertex ("leaf"), and the latter (a, b, c, f, and i) is referred to as a non-terminal vertex. An uppermost non-terminal vertex (a) is referred to as a "root". Among two vertices directly connected by the line, one closer to the root (upper side in the drawing) is referred to as a "parent", and one closer to the leaf is referred to as a "child". The root is the only vertex without the parent in the tree structure. The vertices other than the root always have only one parent. For example, in Fig. 21, b is a child of a and a parent of c, and d is a child of c.

### First Embodiment

A first embodiment of the present invention is a word representation device that performs word representation of a streamline structure of a flow pattern in a two-dimensional domain.

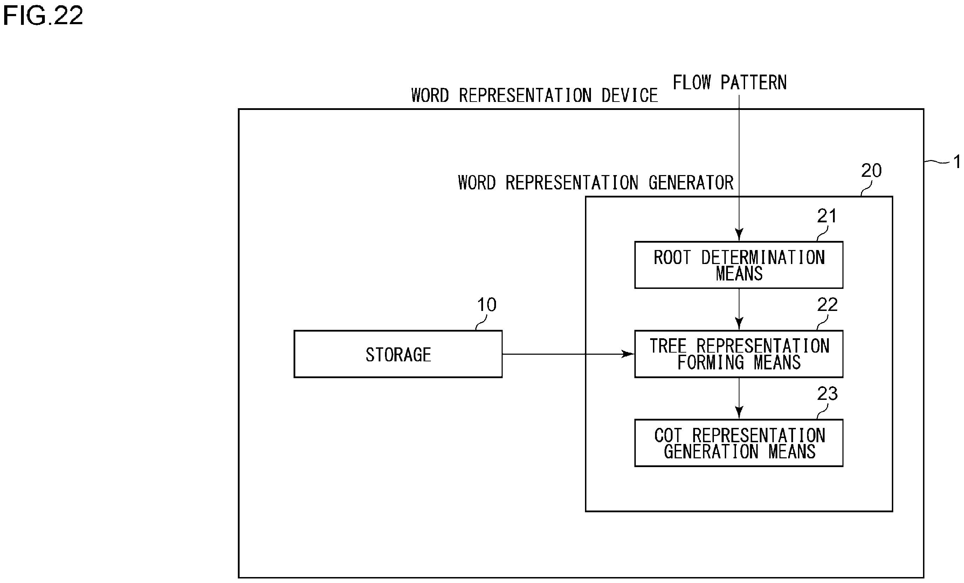

Fig. 22 is a functional block diagram of a word representation device 1 according to the first embodiment. The word representation device 1 is provided with a storage 10 and a word representation generator 20. The word representation generator 20 is provided with a root determination means 21, a tree representation forming means 22, and a COT representation generation means 23.

The storage 10 stores a correspondence relationship between each streamline structure and a character thereof regarding a plurality of streamline structures or vector fields (hereinafter, they are collectively referred to as "streamline structures") forming a finite type flow (hereinafter simply referred to as "flow") pattern.

Among the streamline structures forming the flow pattern, basic structures may be σ_{ϕ±}, σ_{ϕ∼±0}, σ_{ϕ∼±±}, σ_{ϕ∼±}**∓**, β_{ϕ±}, and β_{ϕ2}.

Among the streamline structures forming the flow pattern, two-dimensional structures may be b_{~±} and b_{±}.

Among the streamline structures forming the flow pattern, zero-dimensional point structures may be σ_{±}, σ_{∼±0}, σ_{∼±±}, and σ_{∼±}∓.

Among the streamline structures forming the flow pattern, one-dimensional structures may be p_{~±}, p_{±}, a_{±}, q_{±}, b_{±±}, b_{±}∓, β_{±}, c_{±}, c_{2±}, a₂, γ_{ϕ∼±}, γ_{∼±±}, γ_{-±}∓, a_{∼±}, and q_{~±}.

The root determination means 21 determines a root of a given flow pattern. At that time, a rotational direction of the flow is the rotational direction when a singular orbit or a boundary serving as the root is seen as the center. An "inner structure" of a certain structure refers to a connected component of a complementary set that does not include the root. Furthermore, an "innermost structure" refers to a structure having no inner structure or a structure having no inner structure other than a flow box (a rectangle formed of orbits having a shape of an open interval) as illustrated in Fig. 9(a).

In a case where the structure of the flow corresponding to the root is regarded as a point at infinity or a boundary at infinity, and in a case where there is the structure other than the root, when a certain structure is the innermost structure, the connected component of the complementary set is empty or only the flow box as illustrated in Fig. 9(a). Note that, a "boundary component" refers to a connected component on a boundary.

The root is any one of the followings.
1. The point of infinity being a center "around which orbits are counterclockwise"
2. The point of infinity being a center "around which orbits are clockwise"
3. The point of infinity being a sink "around which orbits do not rotate"
4. The point of infinity being a sink "around which orbits are counterclockwise"
5. The point of infinity being a sink "around which orbits are clockwise"
6. The point of infinity being a source "around which orbits do not rotate"
7. The point of infinity being a source "around which orbits are counterclockwise"
8. The point of infinity being a source "around which orbits are clockwise"
9. A boundary component formed of counterclockwise orbits as seen from the point of infinity and a α-saddle (in this case, there is no α-saddle having a separatrix connected to a source structure in a child of the root. In this case, assuming that the point of infinity is included in the boundary component serving as the root, as seen from an origin located at □_{bϕ∼-}, the boundary serving as the root seems to rotate clockwise).
10. A boundary component formed of clockwise orbits as seen from the point of infinity and a α-saddle (in this case, there is no α-saddle having a separatrix connected to a source).
11. A α-saddle having a separatrix connected to a sink, a α-saddle having a separatrix connected to a source adjacent thereto in a counterclockwise direction, and a boundary component.

Note that, in a case of determining a rotational direction of the sink/source at the point of infinity from finite data, it is possible to collapse a boundary of a data domain into one point and determine the rotational direction considering that point as the point of infinity. For example, it is possible to set a threshold δ and a probability p, and determine the rotational direction with reference to a criterion of determining to rotate in a (counter)clockwise direction in a case where a ratio of "vectors brought into contact with the boundary in the (counter)clockwise direction with an angular error of δ or smaller" is not smaller than the probability p, and determining not to rotate in other cases.

A symbol of the root is σ_{ϕ+}(□_{bϕ}-) in a case of 1, σ_{ϕ-}(□_{bϕ+}) in a case of 2, σ_{ϕ∼-0}(□_{bϕ∼+}) in a case of 3, σ_{ϕ∼-+}(□_{bϕ∼+}) in a case of 4, σ_{ϕ~--}(□_{bϕ∼+}) in a case of 5, σ_{ϕ∼+0}(□_{bϕ∼-}) in a case of 6, σ_{ϕ∼++}(□_{bϕ∼-}) in a case of 7, σ_{ϕ∼+-} (□_{bϕ∼-}) in a case of 8, β_{ϕ+}(□_{bϕ∼-}, {□_{c+s}}) in a case of 9, β_{ϕ-}(□_{bϕ∼+}, {□_{c-s}}) in a case of 10, and β_{2ϕ}({□_{c+s}, □_{-∓}, □_{c-s}, □_{∼-}, □_{γϕs}}, □ₐₛ)in a case of 11.

When the root is determined, the direction of the flow such as a periodic orbit is determined as follows, and a sign of a local structure of the flow is determined.
1. The root is regarded as the point of infinity or the boundary of infinity, and a remaining structure is regarded to be on a plane.
2. Looking at a direction of the structure of the flow to be extracted, the sign is determined to be + in a case of the counterclockwise rotation, and determined to be - in a case of the clockwise rotation.

Furthermore, signs ~±± and ~±∓ regarding the direction and sink/source are determined as follows.
1. When the structure of the flow is extracted, in a case where a parent structure thereof is the sink structure and a non-closed orbit rotates counterclockwise in the vicinity of the same, the sign is set to ~-+.
2. When the structure of the flow is extracted, in a case where a parent structure thereof is the sink structure and a non-closed orbit rotates clockwise in the vicinity of the same, the sign is set to ∼--.
3. When the structure of the flow is extracted, in a case where a parent structure thereof is the sink structure and a non-closed orbit does not rotate in the vicinity of the same, the sign is set to ~-0.
4. When the structure of the flow is extracted, in a case where a parent structure thereof is the source structure and a non-closed orbit rotates counterclockwise in the vicinity of the same, the sign is set to ~++.
5. When the structure of the flow is extracted, in a case where a parent structure thereof is the source structure and a non-closed orbit rotates counterclockwise in the vicinity of the same, the sign is set to ~+-.
6. When the structure of the flow is extracted, in a case where a parent structure thereof is the source structure and a non-closed orbit does not rotate in the vicinity of the same, the sign is set to ~+0.

The tree representation forming means 22 repeatedly executes processing of extracting the streamline structure of the given flow pattern, assigning a character to the extracted streamline structure on the basis of the correspondence relationship stored in the storage 10, and deleting the extracted streamline structure from the innermost portion of the flow pattern until reaching the root, thereby forming a tree representation of the given flow pattern. The formation of the tree representation is executed on the basis of the following principles.
1. The streamline structure is extracted from the given flow pattern.
2. When the streamline structure is extracted, the character (COT representation) corresponding to the streamline structure is assigned as a vertex of the tree. Then, the streamline structure is deleted. Hereinafter, the processing of "extracting the streamline structure from the flow pattern, assigning the character to the streamline structure, and deleting the streamline structure" is collectively represented as "extracting the structure".
3. When extracting the structure, it starts from extracting the innermost structure, and the structure is sequentially extracted until all the structures are removed.
   3.1. The innermost structure corresponds to a leaf.
   3.2. The structure extracted last corresponds to the root. That is, the processing of extracting the structure is repeated until reaching the root of the given flow pattern.
   3.3. When extracting a certain structure, the structure is replaced with □, and □ and the structure are linked so as to correspond (by this, when extracting an upper structure including this □, this structure may be made a "child" structure of the upper structure).

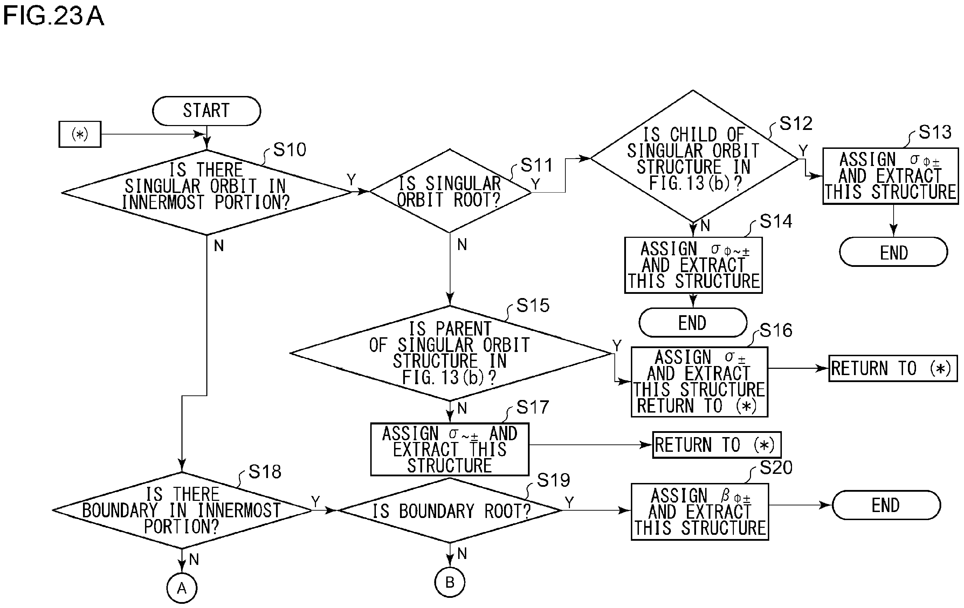

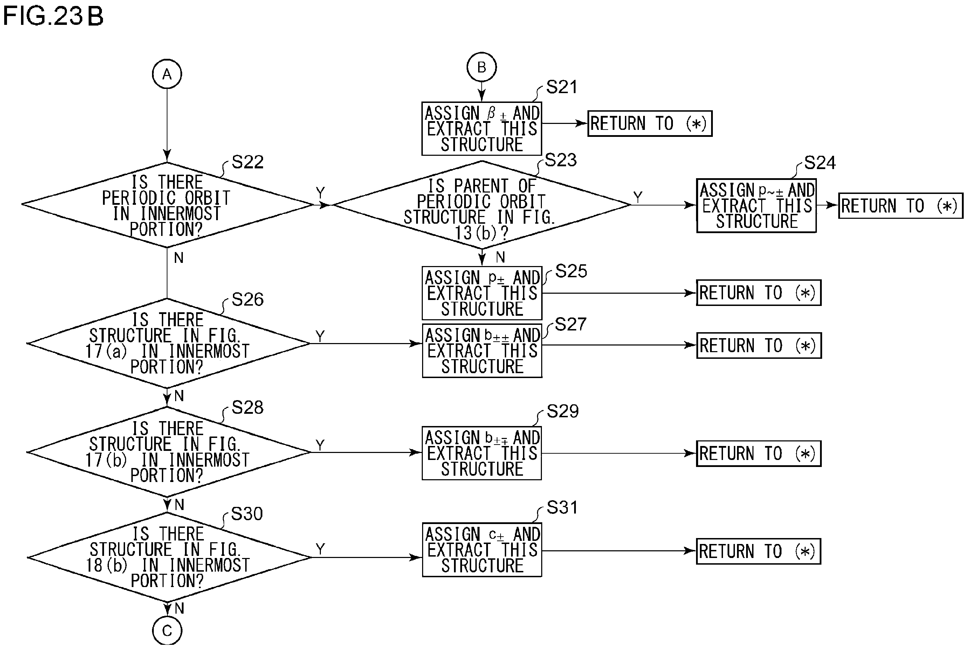

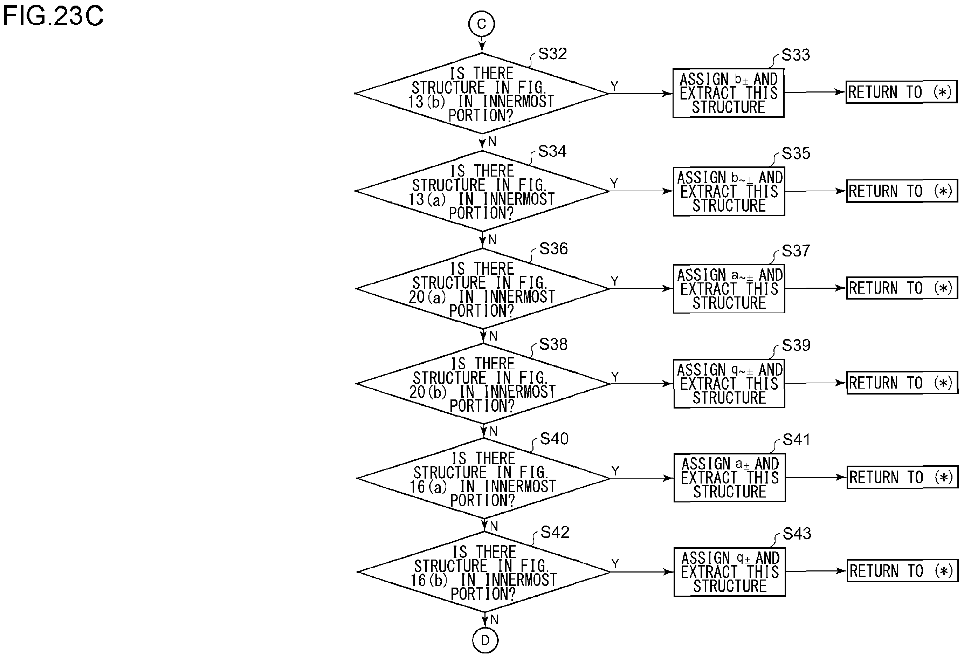

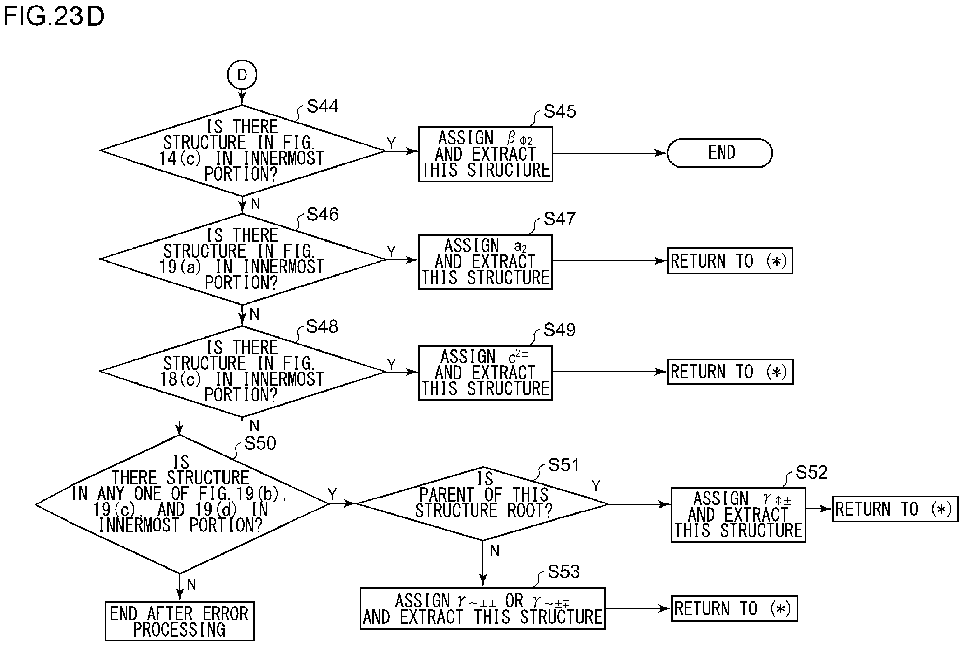

Hereinafter, processing in which the tree representation forming means 22 forms the tree representation is described in detail with reference to Fig. 23. The tree representation forming means 22 forms the tree representation by sequentially adding vertices from the leaf toward the root. Specifically, in the process of extracting from the structure of the innermost flow, the tree representation is formed by adding the vertex to the leaf. That is, the tree representation is formed by adding a new vertex while confirming two points of "what is a child of the vertex to be newly added" and "arrangement thereof".

Specifically, the tree representation forming means 22 forms the tree representation by executing processing at steps S10 to S53. Fig. 23 is a flowchart illustrating the processing at steps S10 to S53. Fig. 23(a) illustrates a flow at steps S10 to S20. Fig. 23(b) illustrates a flow at steps S21 to S31. Fig. 23(c) illustrates a flow at steps S32 to S43. Fig. 23(d) illustrates a flow at steps S44 to S53. As described above, the formation of the tree representation starts in a state in which the root is determined in the given flow pattern.

At step S10, this method determines whether there is a singular orbit in the innermost portion of the given flow pattern. In a case where the determination at step S10 is positive, the procedure shifts to step S11. On the other hand, in a case where this is negative, the procedure shifts to step S18.

At step S11, this method determines whether a specific point present in the innermost portion is the root. In a case where the determination at step S11 is positive, the procedure shifts to step S12. On the other hand, in a case where this is negative, the procedure shifts to step S15.

At step S12, this method determines whether a structure of a child of the singular orbit described above is a structure illustrated in Fig. 13(b). In a case where the determination at step S12 is positive, the procedure shifts to step S13. On the other hand, in a case where this is negative, the procedure shifts to step S14.

At step S13, this method assigns a character σ_{ϕ±} to the above-described singular orbit and extracts this singular orbit from the flow pattern. When all the singular orbits are extracted, the procedure ends.

At step S14, this method assigns any one of characters σ_{ϕ∼±0}, σ_{ϕ∼±±,} and σ_{ϕ∼±}∓ to the above-described singular orbit according to the rotational direction, and extracts the singular orbit from the flow pattern. That is, around the source/sink that is the point at the point of infinity, when a non-closed orbit group does not rotate, σ_{ϕ∼±0} is assigned, when this rotates counterclockwise, σ_{ϕ∼}∓₊ is assigned, and when this rotates clockwise, σ_{ϕ∼}∓₋ is assigned all in double-sign in same order, and this singular orbit is extracted from the flow pattern. In a case where there is a plurality of same singular orbits, processing of assigning characters σ_{ϕ∼±0}, σ_{ϕ∼±±,} and σ_{ϕ∼±}∓ to the respective singular orbits and extracting the same from the flow pattern is repeated. When all the singular orbits are extracted, the procedure ends.

At step S15, this method determines whether a structure of a parent of the singular orbit described above is the structure illustrated in Fig. 13(b). In a case where the determination at step S15 is positive, the procedure shifts to step S16. On the other hand, in a case where this is negative, the procedure shifts to step S17.

At step S16, this method assigns a character σ_{±} to the above-described singular orbit and extracts this singular orbit from the flow pattern. In a case where there is a plurality of same singular orbits, processing of assigning the character σ_{±} to the respective singular orbits and extracting the same from the flow pattern is repeated. When all the singular orbits are extracted, the procedure returns to step S10.

At step S17, this method assigns any one of characters σ_{~±0}, σ_{∼±±}, and σ_{-±}∓ to the above-described singular orbit according to the rotational direction, and extracts the singular orbit from the flow pattern. That is, around the source/sink, when a non-closed orbit group does not rotate, σ_{~±0} is assigned, when this rotates counterclockwise, σ_{~}∓₊ is assigned, and when this rotates clockwise, _{σ~}∓₋ is assigned all in double-sign in same order, and this singular orbit is extracted from the flow pattern. In a case where there is a plurality of same singular orbits, processing of assigning the characters σ_{∼±0}, σ_{∼±±}, and σ_{∼±}∓ to the respective singular orbits and extracting the same from the flow pattern is repeated. When all the singular orbits are extracted, the procedure returns to step S10.

At step S18, this method determines whether there is a boundary in the innermost portion of the given flow pattern. In a case where the determination at step S18 is positive, the procedure shifts to step S19. On the other hand, in a case where this is negative, the procedure shifts to step S22.

At step S19, this method determines whether the boundary in the innermost portion is the root. In a case where the determination at step S19 is positive, the procedure shifts to step S20. On the other hand, in a case where this is negative, the procedure shifts to step S21.

At step S20, this method assigns a character β_{ϕ±} to the above-described boundary and extracts this boundary from the flow pattern. When all the boundaries are extracted, the procedure ends.

At step S21, this method assigns a character β_{±} to the above-described boundary and extracts this boundary from the flow pattern. In a case where there is a plurality of same boundaries, processing of assigning the character β_{±} to the respective boundaries and extracting the same from the flow pattern is repeated. When all the boundaries are extracted, the procedure returns to step S10.

At step S22, this method determines whether there is a periodic orbit in the innermost portion of the given flow pattern. In a case where the determination at step S22 is positive, the procedure shifts to step S23. On the other hand, in a case where this is negative, the procedure shifts to step S26.

At step S23, this method determines whether a structure of a parent of the periodic orbit described above is the structure illustrated in Fig. 13(b). In a case where the determination at step S23 is positive, the procedure shifts to step S24. On the other hand, in a case where this is negative, the procedure shifts to step S25.

At step S24, this method assigns a character p_{~±} to the above-described periodic orbit and extracts this periodic order from the flow pattern. In a case where there is a plurality of same periodic orbits, processing of assigning the character p_{~±} to the respective periodic orbits and extracting the same from the flow pattern is repeated. When all the periodic orbits are extracted, the procedure returns to step S10.

At step S25, this method assigns a character p_{±} to the above-described periodic orbit and extracts this periodic order from the flow pattern. In a case where there is a plurality of same periodic orbits, processing of assigning the character p_{±} to the respective periodic orbits and extracting the same from the flow pattern is repeated. When all the periodic orbits are extracted, the procedure returns to step S10.

At step S26, this method determines whether there is a structure illustrated in Fig. 17(a) in the innermost portion of the given flow pattern. In a case where the determination at step S26 is positive, the procedure shifts to step S27. On the other hand, in a case where this is negative, the procedure shifts to step S28.

At step S27, this method assigns a character b_{±±} to the structure illustrated in Fig. 17(a) described above and extracts this structure from the flow pattern. In a case where there is a plurality of same structures, processing of assigning the character b_{±±} to the respective structures and extracting the same from the flow pattern is repeated. When all the structures illustrated in Fig. 17(a) are extracted, the procedure returns to step S10.

At step S28, this method determines whether there is a structure illustrated in Fig. 17(b) in the innermost portion of the given flow pattern. In a case where the determination at step S28 is positive, the procedure shifts to step S29. On the other hand, in a case where this is negative, the procedure shifts to step S30.

At step S29, this method assigns a character b_{±}∓ to the structure illustrated in Fig. 17(b) described above and extracts this structure from the flow pattern. In a case where there is a plurality of same structures, processing of assigning the character b_{±}∓ to the respective structures and extracting the same from the flow pattern is repeated. When all the structures illustrated in Fig. 17(b) are extracted, the procedure returns to step S10.

At step S30, this method determines whether there is a structure illustrated in Fig. 18(b) in the innermost portion of the given flow pattern. In a case where the determination at step S30 is positive, the procedure shifts to step S31. On the other hand, in a case where this is negative, the procedure shifts to step S32.

At step S31, this method assigns a character c_{±} to the structure illustrated in Fig. 18(b) described above and extracts this structure from the flow pattern. In a case where there is a plurality of same structures, processing of assigning the character c_{±} to the respective structures and extracting the same from the flow pattern is repeated. When all the structures illustrated in Fig. 18(b) are extracted, the procedure returns to step S10.

At step S32, this method determines whether there is a structure illustrated in Fig. 13(b) in the innermost portion of the given flow pattern. In a case where the determination at step S32 is positive, the procedure shifts to step S33. On the other hand, in a case where this is negative, the procedure shifts to step S34.

At step S33, this method assigns a character b_{±} to the structure illustrated in Fig. 13(a) described above and extracts this structure from the flow pattern. In a case where there is a plurality of same structures, processing of assigning the character b_{±} to the respective structures and extracting the same from the flow pattern is repeated. When all the structures illustrated in Fig. 13(b) are extracted, the procedure returns to step S10.

At step S34, this method determines whether there is a structure illustrated in Fig. 13(a) in the innermost portion of the given flow pattern. In a case where the determination at step S34 is positive, the procedure shifts to step S35. On the other hand, in a case where this is negative, the procedure shifts to step S36.

At step S35, this method assigns a character b_{~±} to the structure illustrated in Fig. 13(a) described above and extracts this structure from the flow pattern. In a case where there is a plurality of same structures, processing of assigning the character b_{~±} to the respective structures and extracting the same from the flow pattern is repeated. When all the structures illustrated in Fig. 13(a) are extracted, the procedure returns to step S10.

At step S36, this method determines whether there is a structure illustrated in Fig. 20(a) in the innermost portion of the given flow pattern. In a case where the determination at step S36 is positive, the procedure shifts to step S37. On the other hand, in a case where this is negative, the procedure shifts to step S38.

At step S37, this method assigns a character a_{~±} to the structure illustrated in Fig. 20(a) described above and extracts this structure from the flow pattern. In a case where there is a plurality of same structures, processing of assigning the character a_{~±} to the respective structures and extracting the same from the flow pattern is repeated. When all the structures illustrated in Fig. 20(a) are extracted, the procedure returns to step S10.

At step S38, this method determines whether there is a structure illustrated in Fig. 20(b) in the innermost portion of the given flow pattern. In a case where the determination at step S38 is positive, the procedure shifts to step S39. On the other hand, in a case where this is negative, the procedure shifts to step S40.

At step S39, this method assigns a character q~± to the structure illustrated in Fig. 20(b) described above and extracts this structure from the flow pattern. In a case where there is a plurality of same structures, processing of assigning the character q~± to the respective structures and extracting the same from the flow pattern is repeated. When all the structures illustrated in Fig. 20(b) are extracted, the procedure returns to step S10.

At step S40, this method determines whether there is a structure illustrated in Fig. 16(a) in the innermost portion of the given flow pattern. In a case where the determination at step S40 is positive, the procedure shifts to step S41. On the other hand, in a case where this is negative, the procedure shifts to step S42.

At step S41, this method assigns a character a_{±} to the structure illustrated in Fig. 16(a) described above and extracts this structure from the flow pattern. In a case where there is a plurality of same structures, processing of assigning the character a_{±} to the respective structures and extracting the same from the flow pattern is repeated. When all the structures illustrated in Fig. 16(a) are extracted, the procedure returns to step S10.

At step S42, this method determines whether there is a structure illustrated in Fig. 16(b) in the innermost portion of the given flow pattern. In a case where the determination at step S42 is positive, the procedure shifts to step S43. On the other hand, in a case where this is negative, the procedure shifts to step S44.

At step S43, this method assigns a character q_{±} to the structure illustrated in Fig. 16(b) described above and extracts this structure from the flow pattern. In a case where there is a plurality of same structures, processing of assigning the character q_{±} to the respective structures and extracting the same from the flow pattern is repeated. When all the structures illustrated in Fig. 16(b) are extracted, the procedure returns to step S10.

At step S44, this method determines whether there is a structure illustrated in Fig. 14(c) in the innermost portion of the given flow pattern. In a case where the determination at step S44 is positive, the procedure shifts to step S45. On the other hand, in a case where this is negative, the procedure shifts to step S46.

At step S45, this method assigns a character β_{φ2} to the structure illustrated in Fig. 14(c) described above and extracts this structure from the flow pattern. When all the boundaries are extracted, the procedure ends.

At step S46, this method determines whether there is a structure illustrated in Fig. 19(a) in the innermost portion of the given flow pattern. In a case where the determination at step S46 is positive, the procedure shifts to step S47. On the other hand, in a case where this is negative, the procedure shifts to step S48.

At step S47, this method assigns a character a₂ to the structure illustrated in Fig. 19(a) described above and extracts this structure from the flow pattern. In a case where there is a plurality of same structures, processing of assigning the character a₂ to the respective structures and extracting the same from the flow pattern is repeated. When all the structures illustrated in Fig. 19(a) are extracted, the procedure returns to step S10.

At step S48, this method determines whether there is a structure illustrated in Fig. 18(c) in the innermost portion of the given flow pattern. In a case where the determination at step S48 is positive, the procedure shifts to step S49. On the other hand, in a case where this is negative, the procedure shifts to step S50.

At step S49, this method assigns a character c_{2±} to the structure illustrated in Fig. 18(c) described above and extracts this structure from the flow pattern. In a case where there is a plurality of same structures, processing of assigning the character c_{2±} to the respective structures and extracting the same from the flow pattern is repeated. When all the structures illustrated in Fig. 18(c) are extracted, the procedure returns to step S10.

At step S50, this method determines whether there is any one of structures illustrated in Figs. 19(b), 19(c), and 19(d) in the innermost portion of the given flow pattern. In a case where the determination at step S50 is positive, the procedure shifts to step S51. On the other hand, in a case where this is negative, it is not possible to assign a character to the structure, so that the procedure ends after performing error processing.

At step S51, this method determines whether a parent of any one of the structures illustrated in Figs. 19(b), 19(c), and 19(d) described above is the root. In a case where the determination at step S51 is positive, the procedure shifts to step S52. On the other hand, in a case where this is negative, the procedure shifts to step S53.

At step S52, this method assigns a character γ_{ϕ∼±} to the structure illustrated in Fig. 19(b) described above and extracts this structure from the flow pattern. In a case where there is a plurality of same structures, processing of assigning the character γ_{ϕ∼±} to the respective structures and extracting the same from the flow pattern is repeated. When all the structures illustrated in Fig. 19(b) are extracted, the procedure returns to step S10.

At step S53, this method assigns a character γ_{∼±±} or γ_{∼±}∓ to any one of the structures illustrated in Figs. 19(c) and 19(d) described above and extracts this structure from the flow pattern. In a case where there is a plurality of same structures, processing of assigning the character γ_{∼±±} or _{γ~±}∓ to the respective structures and extracting the same from the flow pattern is repeated. When all the structures illustrated in Figs. 19(c) and 19(d) are extracted, the procedure returns to step S10.

By executing the processing at steps S10 to S53 described above, the tree representation forming means 22 assigns the characters to the flow pattern on an arbitrary curved surface S and obtains an abstract tree representation of the flow pattern.

The abstract tree representation of the flow pattern obtained as described above includes a structure as an abstract graph of an ss-saddle connection diagram; however, in order to convert the same into the COT representation, a combinatorial structure as a curved surface graph of the ss-saddle connection diagram is required, so that a required combinatorial structure of the combinatorial structure as the curved surface graph of the ss-saddle connection diagram is extracted.

For example, a method of extracting the combinatorial structure as the curved surface graph of the saddle connection diagram is as follows.
1. A saddle is extracted.
2. A separatrix connecting the saddles to each other is extracted.
3. An abstract graph obtained from these pieces of information is the abstract graph of the saddle connection diagram, and the saddle connection diagram is formed as the curved surface graph by arranging vertex and sides so as to correspond to arrangement on the curved surface.

By executing the above-described processing, the characters are assigned to the flow pattern on an arbitrary curved surface S to obtain the tree representation of the flow pattern.

The COT representation generation means 23 converts the tree representation formed by the tree representation forming means 22 into the COT representation. Specifically, the COT representation is formed by converting the tree representation into a representation using parentheses. Note that, the conversion is realized by using curly brackets {} in a case of having elements arranged in a circle order, and by using parentheses () in a case of having elements arranged in a total order. Generation of the COT representation is described below by way of illustration.

According to this embodiment, when an arbitrary finite type flow pattern in a two-dimensional domain is given, it is possible to obtain the COT representation of the flow pattern, that is, characterize the flow pattern.

Hereinafter, a procedure of giving the word representation to a specific flow pattern is described.

### First Example of First Embodiment

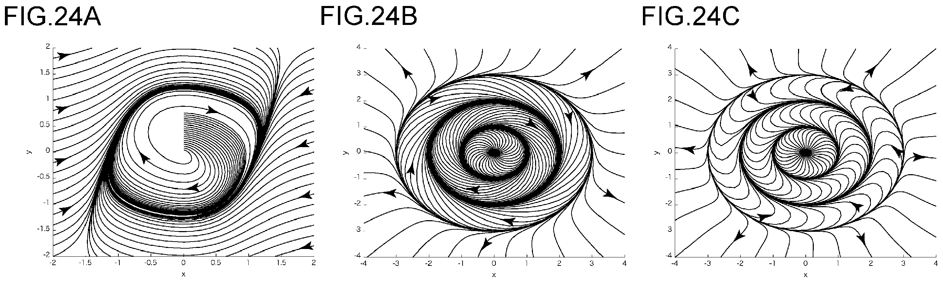

Fig. 24(a) illustrates an example of the flow pattern. It is illustrated that the COT representation of the flow pattern is obtained by executing the following procedure according to the first embodiment.

Procedure:
1. Since there is no physical boundary, the root is the point of infinity. Since the point of infinity is the source and the non-closed orbit rotates counterclockwise in the vicinity of the point of infinity, the character corresponding to the root is σ_{ϕ∼++}(□_{bϕ∼-}). Then, the root is determined.
2. Since the innermost portion is the source and the non-closed orbit rotates clockwise in the vicinity thereof, the corresponding character is σ_{∼+-}. Therefore, this structure is first extracted and replaced with □_{∼+}. The word generated at that time is b_{~+}(σ_{~+-}, λ_{∼+}).
3. Since the innermost portion becomes an open annular domain by a source flow, the corresponding character is b_{~+}(□_{∼+}, {□_{a~+s}}). This structure is extracted and replaced with □_{b+}. The word generated at that time is p₋(b_{~+}(σ_{~+-}, λ_{∼+})).
4. Since the innermost portion is a clockwise periodic orbit, the corresponding character is p₋(□b₋). This structure is extracted and replaced with □∼-. The word generated at that time is b_{∼-}(p₋(b_{~+}(a_{~+-}, λ_{∼+})), λ_{∼-}).
5. Since the innermost portion becomes the open annular domain by a sink flow, the corresponding character is b_{∼-}(□_{∼-}, {□_{a∼-s}}). This structure is extracted and replaced with □_{bϕ∼-}. The word generated at that time is σ_{ϕ∼++}(b_{∼-}(p₋(b_{∼+}(σ_{~+-}, λ_{∼+})), λ_{∼-})).
6. The innermost portion is the root, and the corresponding character is σ_{ϕ∼+-}(□_{bϕ∼-}). The generated word is σ_{ϕ∼++}(b_{∼-}(p₋(b_{∼+}(σ_{∼+-}, λ_{∼+})), λ_{∼-})).

It is described that the COT representation σ_{ϕ∼++}(b_{∼-}(p₋(b_{∼+}(σ_{∼+-}, λ_{∼+})), λ_{∼-}))for the flow pattern in Fig. 24(a) is given by executing the above-described procedure.

### Second Example of First Embodiment

Fig. 24(b) illustrates another example of the flow pattern. It is illustrated that the COT representation of the flow pattern is obtained by executing the following procedure according to the first embodiment.

Procedure:
1. Since there is no physical boundary, the root is the point of infinity. Since the point of infinity is the sink and the non-closed orbit rotates counterclockwise in the vicinity of the point of infinity, the character corresponding to the root is σϕ_{∼-+}(□_{bϕ∼+}). Then, the root is determined.
2. Since the innermost portion is the sink and the non-closed orbit rotates clockwise in the vicinity thereof, the corresponding character is σ_{∼--}. This structure is extracted and replaced with □_{∼-}. The word generated at that time is σ_{∼--}.
3. Since the innermost portion becomes an open annular domain by a sink flow, the corresponding character is b_{∼-}(□_{∼-}, {□_{a∼-s}}). This structure is extracted and replaced with □_{b-}. The word generated at that time is b_{∼-}(σ_{∼--}, λ_{∼-}).
4. Since the innermost portion is a clockwise periodic orbit, the corresponding character is p₋(□_{b-}). This structure is extracted and replaced with □_{∼-}. The word generated at that time is p₋(b_{~-}(σ_{~--}, λ_{∼-})).
5. Since the innermost portion becomes the open annular domain by a source flow, the corresponding character is b_{~+}(□_{∼+}, {□_{a~+s}}). This structure is extracted and replaced with □_{b+}. The word generated at that time is b_{~+}(p₋(b_{∼-}(a_{∼--}, λ_{∼-}))).
6. Since the innermost portion is a clockwise periodic orbit, the corresponding character is p₋(□_{b-}). This structure is extracted and replaced with □_{∼-}. The word generated at that time is p₋(b_{~+}(p₋(b_{∼-}(σ_{∼--}, λ_{∼-})), λ_{∼-})).
7. Since the innermost portion becomes the open annular domain by the sink flow, the corresponding character is b_{∼-}(□_{∼-}, {□_{a~-s}}). This structure is extracted and replaced with □_{b-}. The word generated at that time is b_{∼-}(p₋(b_{~+}(p₋(b_{∼-}(σ_{∼--}, λ_{∼-})), λ_{∼-})), λ_{∼-}).
8. Since the innermost portion is the clockwise periodic orbit, the corresponding character is p₋(□_{b-}). This structure is extracted and replaced with □_{∼-}. The word generated at that time is p₋(b_{~-}(p₋(b_{∼+}(p₋,(b_{∼-},(σ_{∼--}, λ_{∼-})), λ_{∼-})), λ_{∼-})) .
9. Since the innermost portion becomes the open annular domain by the source flow, the corresponding character is b_{~+}(□_{∼+}, {□_{a~+s}}). This structure is extracted and replaced with □_{b+}. The word generated at that time is b_{~+}(p₋(b_{∼-}(p₋(b_{~+}(p₋(b_{∼-}(σ_{∼--,} λ_{∼-})), λ_{∼-})), λ_{∼-})), {□_{a~-s}}).
10. The innermost portion is the root, and the corresponding character is σ_{ϕ∼-+}(□_{bϕ∼+}). The generated word is σ_{ϕ∼-+}(b_{∼+}(p₋(b_{∼-}(p₋(b_{~+}(p₋(b_{∼-}(σ_{∼--}, λ_{∼-})), λ_{∼-})), λ_{∼-})), λ_{∼-})).

It is described that the COT representation σ_{ϕ∼-+}(b_{∼+}(p₋(b_{∼-}(p₋(b_{∼+}(p₋(b_{∼-}(σ_{∼--}, λ_{∼-})), λ_{∼-})), λ_{∼-}, )for the flow pattern in Fig. 24(b) is given by executing the above-described procedure.

### Third Example of First Embodiment

Fig. 24(c) illustrates another example of the flow pattern. It is illustrated that the COT representation of the flow pattern is obtained by executing the following procedure according to the first embodiment.

Procedure:
1. Since there is no physical boundary, the root is the point of infinity. Since the point of infinity is the sink and the non-closed orbit rotates clockwise in the vicinity of the point of infinity, the character corresponding to the root is σ_{ϕ∼--}(□_{bϕ∼+}). Then, the root is determined.
2. Since the innermost portion is the sink and the non-closed orbit rotates counterclockwise in the vicinity thereof, the corresponding character is σ_{∼-+}. This structure is extracted and replaced with _{□∼-}. The word generated at that time is σ_{∼-+}.
3. Since the innermost portion becomes an open annular domain by a sink flow, the corresponding character is b_{∼-}(□_{∼-}, {□_{a∼-s}}). This structure is extracted and replaced with □_{b-}. The word generated at that time is b_{∼-}(β_{∼-+}, λ_{∼-}).
4. Since the innermost portion is a counterclockwise periodic orbit, the corresponding character is p₊(□_{b+}). This structure is extracted and replaced with _{□∼+}. The word generated at that time is p₊(b_{∼-}(σ_{∼-+})).
5. Since the innermost portion becomes the open annular domain by a source flow, the corresponding character is b_{~+}(□_{∼+}, {□_{a~+s}}). This structure is extracted and replaced with □_{b+}. The word generated at that time is b_{~+}(p₊(b_{∼-}(σ_{∼-+}))).
6. Since the innermost portion is a clockwise periodic orbit, the corresponding character is p₋(□_{b-}). This structure is extracted and replaced with _{□∼-}. The word generated at that time is p₋(b_{∼+}(p₊(b_{∼-}(σ_{∼-+}, λ_{∼-})), λ_{∼+})).
7. Since the innermost portion becomes the open annular domain by the sink flow, the corresponding character is b_{∼-}(□_{∼-}, {□_{a~-s}}). This structure is extracted and replaced with □_{b-}. The word generated at that time is b_{∼-}(p₋(b_{~+}(p₊(b_{∼-}(σ_{∼-+}, λ_{∼-})), λ_{∼+})), λ_{∼-}).
8. Since the innermost portion is the counterclockwise periodic orbit, the corresponding character is p₊(□_{b+}). This structure is extracted and replaced with _{□∼+}. The word generated at that time is p₊(b_{∼-}(p₋(b_{∼+}(p₊(b_{∼-}(β_{∼-+}, λ_{∼+})), λ_{∼-})), λ_{∼-})) .
9. Since the innermost portion is the open annular domain by the source flow, the corresponding character is b_{~+}(□_{∼+}, {□_{a~+s}}). This structure is extracted and replaced with □_{b+}. The word generated at that time is b_{~+}(p₊(b_{∼-}(p₋(b_{∼+}(p₊(b_{∼-}(σ_{∼-+}, λ_{∼+})), λ_{∼+})), λ_{∼-})), λ_{∼-}).
10. The innermost portion is the root, and the corresponding character is σ_{ϕ∼-}(□_{bϕ∼+}). The generated word is σ_{ϕ∼--}(b_{∼+}(p₊(b_{∼-}(p₋(b_{∼+}(p₊(b_{∼-}(β_{∼-+}, λ_{∼-})), λ_{∼+})), λ_{∼-})), λ_{∼+})) .

It is described that the COT representation σ_{ϕ∼--}(b_{∼+}(p₊(b_{∼-}(p₋(b_{∼+}(p₊(b_{∼-}(β_{∼-+}, λ_{∼-})), λ_{∼+})), λ_{∼-})), λ_{∼+})) for the flow pattern in Fig. 24(c) is given by executing the above-described procedure.

### Fourth Example of First Embodiment

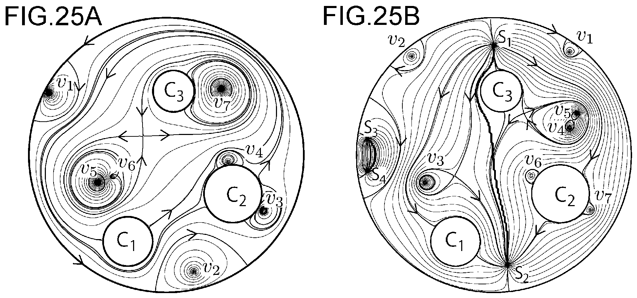

Fig. 25(a) illustrates another example of the flow pattern. It is illustrated that the COT representation of the flow pattern is obtained by executing the following procedure according to the first embodiment.

Procedure:
1. The root is a physical boundary, and the character corresponding to the root is β_{ϕ-}(□_{b+}, {□_{c+s}}). Then, the root is determined.
2. Since the innermost portion is the center, the corresponding character is σ_{±}. This structure is extracted and replaced with □_{±}. At that time, a total of seven words σ₊ and σ₋ are generated corresponding to v₁ to v₇.
3. Since the innermost portion is an open annulus formed of the periodic orbit, the corresponding character is b_{±}(□_{α±}). Furthermore, since a parent is not the periodic orbit, this structure is extracted and replaced with □_{b±}. At that time, a total of seven words of three b+(o+) and four b-(σ₋) are generated corresponding to the open annulus formed of the periodic orbit around v₁ to v₇.
4. Since the innermost portion becomes b+-, this structure is extracted and replaced with □_{α+}. At that time, the word generated corresponding to the structure including v₅ is b₊₋(b₊(σ₊), b₋(σ₋)).
5. Since the innermost portion becomes c_{±}, this structure is extracted and replaced with □_{c±s}. At that time, a total of five words of two c₊(b₊(σ₊), λ_{∼-})and three c-(b-(σ₋), λ₊) are generated corresponding to including v₁ to v₄ and v₇.
6. Since the innermost portion becomes β₊, this structure is extracted and replaced with □_{c±s}. At that time, the word generated corresponding to c₃ is β₊{c₊(b₊(σ₊), λ₋)}.
7. Since the innermost portion is the open annulus formed of the periodic orbit, the corresponding character is b₊(□_{α±}). Furthermore, since the parent is b++, this structure is extracted and replaced with □_{b+}. At that time, the words generated corresponding to the open annulus including vs to v₇ are b₊(b₊₋(b₊(σ₊), b₋(σ₋))) and b₊(β₊{c₊(b₊(σ₊), λ-)}).
8. Since the innermost portion is b++, the corresponding character is b₊₊{□_{b+}, □_{b+}}. The word generated at that time is as follows.
   b₊₊{b₊(b₊₋(b₊(σ₊), b-(o-))), b₊(β₊{c₊(b₊(σ₊), λ-)})}

It is described that the COT representation β_{ϕ-}(b₊(β₊{c₊(b₊(σ₊), λ₋)·c₊(b₊(β₊{c₊(b₊(b₊₊{b₊(b₊₋(b₊(σ₊), b₋(σ₋))), b₊((β₊{c₊(b₊(σ₊), λ₋)})}))}), c₋(b₋(σ₋)))}), {c₊(b₊(σ₊), λ₋)·c₊(b₊(σ₊), λ₋)}) for the flow pattern in Fig. 25 (a) is given by repeating the procedure similar to the above-described procedure.

### Fifth Example of First Embodiment

Fig. 25(b) illustrates another example of the flow pattern. It is illustrated that the COT representation of the flow pattern is obtained by executing the following procedure according to the first embodiment.

Procedure:
1. The root is a physical boundary and S₁, and the character corresponding to the root is β_{ϕ2}({□_{c+s}, □₋₊, □_{c-s}, □_{∼-}, □_{γϕs}}, □as).
2. Since the innermost portion is S₁, S₂, and v₁ to vg, the corresponding characters are σ_{±} and σ_{∼±0}. This structure is extracted and replaced with □_{±} and □_{∼±}.
3. Since the innermost portion is an open annulus formed of the periodic orbit, the corresponding character is b_{±}(□_{α±}). Furthermore, since a parent is not the periodic orbit, this structure is extracted and replaced with □_{b±}. At that time, a total of seven words of three b+(o+) and four b-(σ₋) are generated corresponding to the open annulus formed of the periodic orbit around v₁ to v₇.
4. Since the innermost portion becomes b--, this structure is extracted and replaced with □_{α-}. At that time, the word generated corresponding to the structure including v₄ and v₅ is b₋₋{b₋(σ₋), b₋(σ₋)}.
5. Since the innermost portion is the open annulus formed of the periodic orbit, the corresponding character is b₋(□_{α-}). Furthermore, since the parent is not the periodic orbit, this structure is extracted and replaced with □_{b-}. At that time, the word generated corresponding to the open annulus formed of the periodic orbit around v₄ and v₅ is b-(b-₋{b₋(σ₋), b₋(σ₋)}).
6. Since the innermost portion is c_{±}, the corresponding character is c_{±}(□_{b±}, □_{c}∓ₛ). This structure is extracted and replaced with □_{c±}. At that time, the words generated corresponding to the structure including v₁, v₂, v₆, and v₇ are c₊(b₊(σ₊), λ_{∼-})and c₋(b₋(σ₋), λ₊).
7. Since the innermost portion is a_{±}, the corresponding character is a_{±}(□_{b±}). This structure is extracted, but class-a₂ remains, so that this is not replaced with □. At that time, the words generated corresponding to v₃, v₄ and v₅ are a₊(b₊(σ₊)) and a₋(b₋(b₋₋{b₋(σ₋), b₋(σ₋)})).
8. Since the innermost portion is a₂, the corresponding character is a₂(□_{c+s}, □_{c-s}, □_{γ-s}). This structure is extracted and replaced with □ₐₛ. At that time, the words generated corresponding to the structure including c₁, c₂, and c₃ are a₂(λ₊, λ_{∼-})and a₂(c₊(b₊(σ₊), λ₋), c₋(b₋(σ₋), λ₊)).
9. Since the innermost portion is c₂₋, the corresponding character is c₂₋(□_{c-s}, □_{∼+}, □_{c+s}, □_{∼-}, □_{γ-s}, □_{c-s}, □ₐₛ). This structure is extracted and replaced with □_{c-}. At that time, the word generated corresponding to the structure including s₃ and s₄ is c₂- (λ₊, σ₋₀, λ_{∼-}, σ_{~+0}, λ_{∼-}, λ₊, λ_{∼-}).
10. Herein, since the root is the innermost, the following word is obtained.
   β_{ϕ2}({c₊(b₊(σ₊), λ₋), σ_{∼+0}, c- (b- (σ₋), λ₊) ·c₂₋(λ₊, σ₋₀, λ₋, σ_{~+0}, λ_{∼}, λ₊, λ₋), σ_{~-0}, λ_{∼}}, a₂(λ₊, λ_{∼-})·a₊(b₊(σ₊)) ·a₂(λ₊, λ_{∼-}) ·a₋(b₋(b₋₋{b₋(σ₋), b₋(α₋)}))·a₂(c₊(b₊(σ₊), λ₋), c₋(b₋(σ₋), λ₊))).

It is described that the COT representation β_{ϕ2}({c₊ (b₊ (σ₊), λ₋), σ_{~+0}, c₋ (b₋(σ₋), λ₊)·c₂₋(λ₊, σ₋₀, λ_{∼-}, σ_{~+0}, λ₋, λ₊, λ_{∼}), σ_{∼-0}, λ_{∼}}, a₂(λ₊, λ_{∼-})·a₊(b₊(σ₊))·a₂(λ₊, λ₋) ·a₋(b₋(b₋₋{b₋(σ₋), b₋(σ₋)}))·a₂(c₊(b₊(σ₊), λ₋), c₋(b₋(σ₋), λ₊))) for the flow pattern in Fig. 25(b) is given by executing the above-described procedure.

### Second Embodiment

A second embodiment of the present invention is a word representation device that performs word representation of a streamline structure of a flow pattern in a two-dimensional domain.

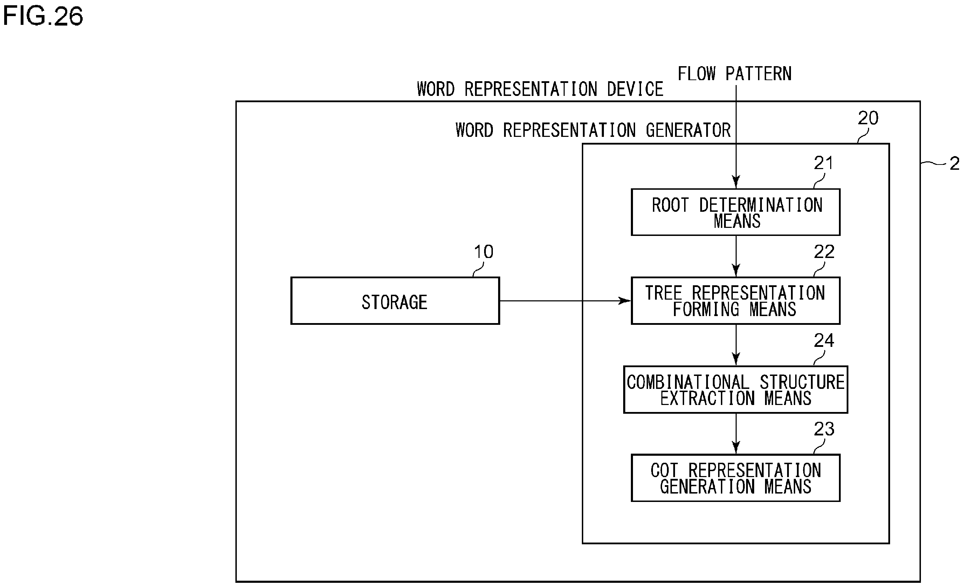

Fig. 26 is a functional block diagram of a word representation device 2 according to the second embodiment. A word representation generator 20 of the word representation device 2 is provided with a combinatorial structure extraction means 24 between a tree representation forming means 22 and a COT representation generation means 23 in addition to a configuration of a word representation device 1 in Fig. 22. Other configurations and operations are the same as those of the word representation device 1.

The combinatorial structure extraction means 24 extracts a combinatorial structure from a given flow pattern. Although the COT representation generated by the word representation device 1 of the first embodiment has a many-to-one correspondence, in the second embodiment, a one-to-one correspondence may be obtained by assigning the combinatorial structure to the COT representation.

The COT representation may uniquely represent an arrangement of a local structure, but does not have one-dimensional global connection information. Therefore, the COT representation is a many-to-one representation. Note that zero-dimensional and two-dimensional structures are uniquely defined by the COT representation. The one-dimensional global connection information is hereinafter referred to as the "combinatorial structure". There is an ss-saddle connection diagram as that having sufficient information of this structure.

The COT representation completely describes the local structure. However, when there are two structures, even if the local structures thereof are the same, entire structures thereof do not necessarily match. Therefore, by assigning the combinatorial structure to the COT representation, the global structure is also determined, and as a result, the structure is determined on a one-to-one basis. However, as described above, the combinatorial structure has only the information of the global connection structure, and does not have any information regarding the zero-dimensional point structure or the two-dimensional structure. Therefore, by combining these two pieces of information, both the local structure and the global structure are determined, and as a result, the structure is determined on a one-to-one basis.

The combinatorial structure extraction means 24 extracts, as a graph structure, to which ss-component an ss-separatrix connected to a saddle is connected. Actually, vertices are "the saddle connected to the ss-component" and "the ss-component connected to the saddle", and a side is the ss-separatrix connected to the saddle. Herein, "the ss-component connected to the saddle" is a structure of any of sink, source, and limit cycle/limit circuit. This graph is an abstract graph in which the number of vertices and sides are finite. In a case where the limit cycle/limit circuit is not included, this abstract graph is realized as a curved surface graph. On the other hand, in a case where the limit cycle/limit circuit is included, since the vertex thereof is realized as a periodic orbit or a circuit, the abstract graph is not realized as the curved surface graph. Therefore, considering a complementary set of the limit cycle/limit circuit and considering a curved surface obtained by collapsing the limit cycle/limit circuit on a newly created boundary into one point, the "ss-component connected to the saddle" includes only the sink and the source, and a new abstract graph obtained by cutting the vertex corresponding to the limit cycle/limit circuit of the abstract graph into two is realized as the curved surface graph. By labeling a set of points obtained by cutting into two and holding information of being cut, the newly obtained curved surface graph has complete information on how the ss-separatrix winds around the ss-component. Therefore, a set of the curved surface graph and the COT representation completely has information of the original ss-saddle connection diagram. Therefore, this curved surface graph is extracted.

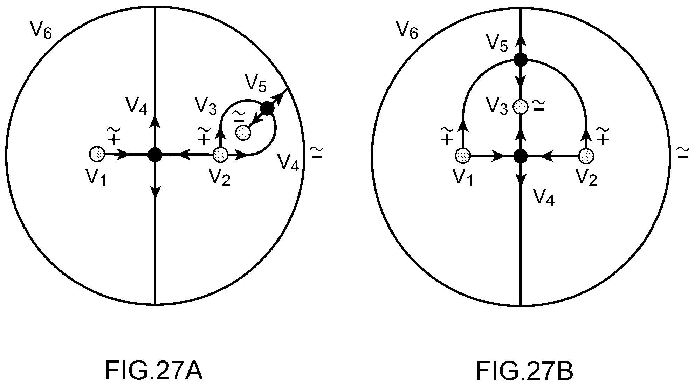

For example, in a case where the limit cycle/limit circuit is not included, there is a following method as a method of extracting the curved surface graph.
1. The saddle connected to the ss-component is extracted.
2. The ss-component connected to the saddle is extracted.
3. The ss-separatrix connected to the saddle is extracted.
4. A planar graph is formed by arranging the vertices and sides of the abstract graph obtained from these pieces of information so as to correspond to arrangement on a plane. For example, Figs. 27(a) and 27(b) are examples of flow patterns having the same COT representation but different streamline topologies due to different global structures (combinatorial structures).

On the other hand, in a case where general limit cycle/limit circuit is included, there is a following method as a method of extracting the curved surface graph.
1. The saddle connected to the ss-component is extracted.
2. The ss-component connected to the saddle is extracted.
3. The ss-separatrix connected to the saddle is extracted.
4. The abstract graph obtained from these pieces of information is obtained.
5. The curved surface obtained by deleting the limit cycle/limit circuit and collapsing the limit cycle/limit circuit on the new boundary into one point is considered.
6. The abstract graph is created by cutting a point corresponding to the limit cycle/limit circuit of the abstract graph into two vertices.
7. Since the limit cycle/limit circuit is not included on the newly obtained curved surface, the curved surface graph is formed by the method of "a case where the limit cycle/limit circuit is not included" described above. Furthermore, to the vertex derived from the limit cycle/limit circuit, information indicating with which point this is originally the same point as a label.

According to this embodiment, when an arbitrary flow pattern on the curved surface is given, one-to-one representation of the flow pattern may be obtained.

### Example of Second Embodiment

Hereinafter, with reference to Fig. 27, a procedure of giving the COT representation to two flow patterns having different streamline topologies is described.

Procedure:
1. There is no physical boundary, and a root is a point of infinity and a sink. A character corresponding to the root is σ_{ϕ-}.
2. First, since an innermost portion is a sink and a source, and a non-closed orbit does not rotate in the vicinity thereof, the corresponding character is σ_{~±0}. Therefore, this structure is extracted and replaced with □_{∼±}. A total of three words of σ_{∼+0} and σ_{∼-ο} are generated at that time.
3. Next, since the innermost portion is a slidable saddle corresponding to a_{~+}, the corresponding character is a_{~+}{□_{~+}, □_{∼+}}. A word generated at that time is a_{~+}{σ_{~+0}, σ_{~+0}}.
4. Next, since the innermost portion is the slidable saddle corresponding to q_{∼-}, the corresponding character is q_{∼-}(□_{∼-}). The word generated at that time is q_{∼-}(σ_{∼-0}).
5. At that time, since the root is in the innermost portion, the following word (COT representation) is obtained.
   σ_{ϕ-}(b_{∼+}(a_{~+}{σ_{~+}, σ_{∼+}}, q_{∼-}(σ_{∼-0})))

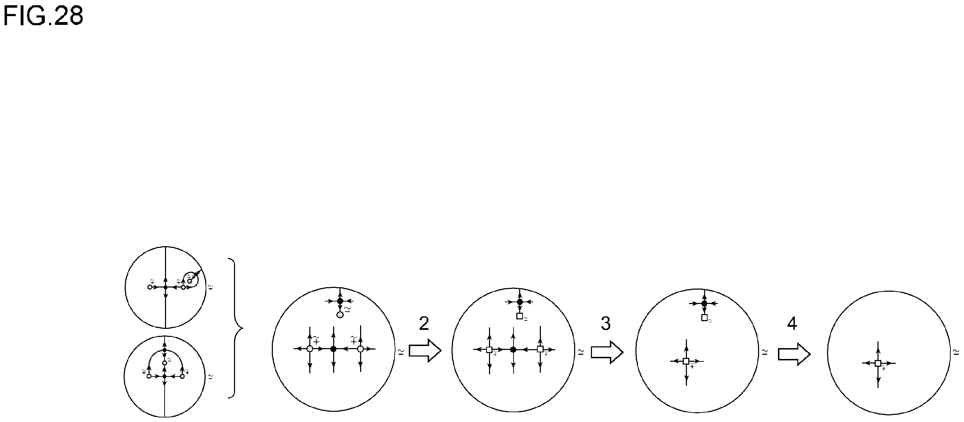

Next, a procedure of extracting the combinatorial structure from the flow patterns illustrated in Figs. 27(a) and 27(b) is described. Fig. 28 illustrates a procedure of extracting the combinatorial structure from the flow pattern in Fig. 27.

Procedure:
1. v₄ and v₅ being the saddles connected to the ss-component are extracted.
2. Two sources v₁ and v₂ being the ss-components connected to the saddle and two sinks v₃ and v₆ are extracted (v₆ is a point of infinity) .
3. By extracting the ss-separatrix connected to the saddle, the curved surface graph that is the combinatorial structure to be extracted is obtained.

By executing this procedure, the combinatorial structure is extracted.

As described above, in a case where the limit cycle/limit circuit is not included, Fig. 28 obtained by extracting the saddle, sink, source, and ss-separatrix is a diagram illustrating the combinatorial structure to be extracted from the flow pattern in Fig. 27.

In a general case, a new curved surface is formed by collapsing the limit cycle/limit circuit on the new boundary into one point in consideration of the complementary set of the limit cycle/limit circuit, and a set of the label of the vertex newly formed by the collapse and the curved surface graph on the curved surface is the combinatorial structure to be extracted from the flow pattern.

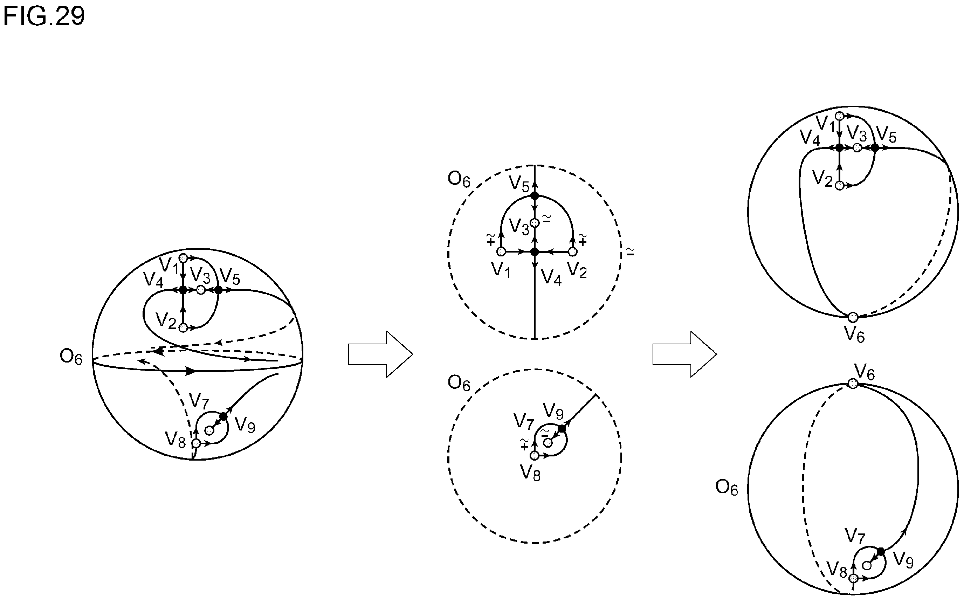

Next, in a case where there is the limit cycle/limit circuit, a procedure of extracting the combinatorial structure illustrated on a right side from the flow pattern illustrated on a left side of Fig. 29 is described.

Procedure:
1. v₄, v₅, and v₉ being the saddles connected to the ss-component are extracted.
2. Three sources v₁, v₂, and v₈ being the ss-components connected to the saddle, two sinks v₃ and v₇, and a limit cycle O₆ are extracted.
3. The limit cycle O₆ is deleted and a complementary set of O₆ illustrated in a center diagram is obtained.
4. Each of two new boundaries is collapsed into one point to obtain a new curved surface illustrated on a right diagram.
5. By extracting the ss-separatrix connected to the saddle, a curved surface graph that is a combinatorial structure to be extracted is obtained.

### Third Embodiment

A third embodiment of the present invention is a word representation method that performs word representation of a streamline structure of a flow pattern in a two-dimensional domain. This method is executed by a computer provided with a storage and a word representation generator.

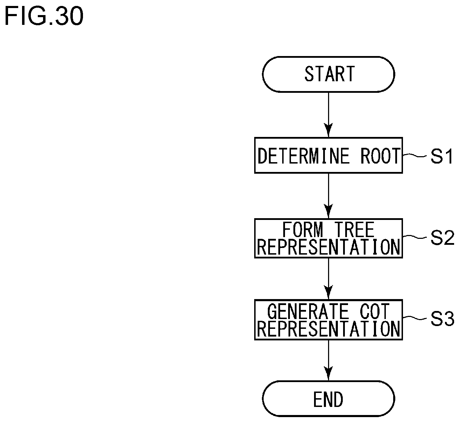

Fig. 30 illustrates a flow of the word representation method according to the third embodiment. The method includes step S1 of determining a root of a given flow pattern, step S2 of forming a tree representation of the flow pattern, and step S3 of generating a COT representation of the flow pattern.

At step S1, the method determines the root of the given flow pattern. Since specific processing of determining the root is the same as that described in the first embodiment, the detailed description thereof is not repeated.

At step S2, the method forms the tree representation of the given flow pattern. Since specific processing of the tree representation formation is the same as that at steps S10 to S53 of the first embodiment, the detailed description thereof is not repeated.

At step S3, this method converts the tree representation formed at step S2 into the COT representation.

According to this embodiment, when an arbitrary flow pattern in the two-dimensional domain is given, it is possible to obtain the COT representation of the flow pattern, that is, characterize the flow pattern.

### Fourth Embodiment

A fourth embodiment of the present invention is a program that allows a computer provided with a storage and a word representation generator to execute processing. This program allows the computer to execute a flow illustrated in Fig. 30. That is, this program allows the computer to execute step S1 of determining a root of a given flow pattern, step S2 of forming a tree representation of the flow pattern, and step S3 of generating a COT representation of the flow pattern.

According to this embodiment, a program that, when an arbitrary flow pattern in a two-dimensional domain is given, obtains the COT representation of the flow pattern, that is, characterizes the flow pattern may be implemented in software, so that highly accurate word representation may be realized using the computer.

### Fifth Embodiment

A fifth embodiment of the present invention is a learning method of a structure shape. Hereinafter, a river is described as an example of a fluid.

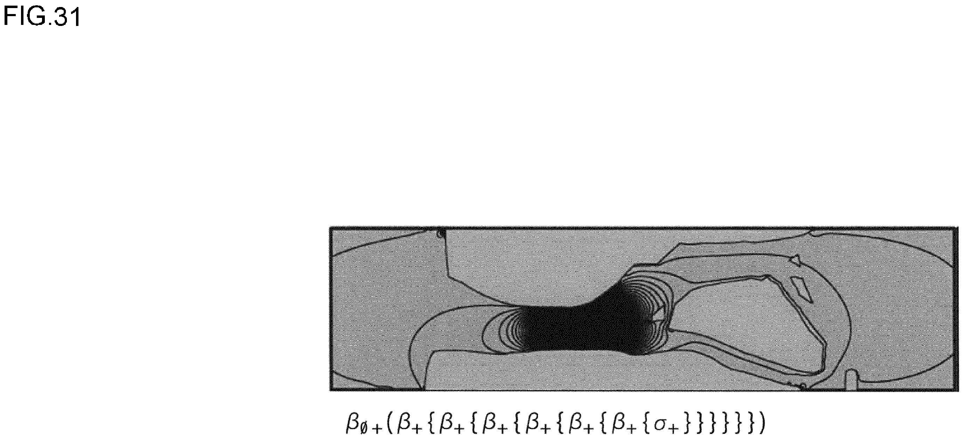

Fig. 31 illustrates a flow pattern obtained by simulation on the basis of a topographic map of the river and a COT representation thereof. As illustrated in Fig. 31, in a place where a depth and a shape of a river bed change, a vortex accompanied with a sink or a source is generated in a flow on a surface of the river. Such vortex might be an obstacle to river traffic, for example. From the viewpoint of safe navigation of ships and disaster prevention, it is desirable to eliminate such vortex and rectify the flow as uniform as possible. However, even if dredging, sediment throwing and the like are performed to eliminate vortex, new sink or source might occur in an unexpected place due to complexity of the shape of the river. An object of this embodiment is to convert a flow pattern generated around a structure into a word representation and allow artificial intelligence (hereinafter, referred to as "AI") to learn a relationship between the word representation and the structure shape, thereby calculating an optimum structure shape for controlling the flow.

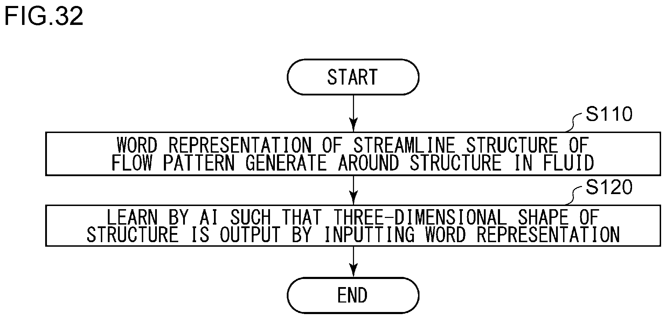

Fig. 32 illustrates a flow of the learning method of the structure shape according to the fifth embodiment. This method includes step S110 of performing word representation of a streamline structure of the flow pattern generated around the structure in the fluid, and step S120 of learning by the AI such that a three-dimensional shape of the structure is output using the word representation as an input.

At step S110, this method performs the word representation of the streamline structure of the flow pattern generated around the structure in the fluid. The fluid is, for example, the river and the like, and the structure is, for example, a river bed, a river bank, a reef and the like of the river. The word representation is a COT representation obtained by inputting the flow pattern to a word representation device 1 in Fig. 22, for example.

At step S120, this method learns by the AI such that the three-dimensional shape of the structure is output by using the word representation as the input. A specific method of the AI is not especially limited, but for example, a neural network such as a convolutional neural network (CNN), a recurrent neural network (RNN), or a long short term memory (LSTM) network may be used, and in this case, different neural networks may be mixed for each calculation model with a common input layer. In this embodiment, a large number of sets of the three-dimensional shape of the structure and the word representation of the flow pattern are prepared, and the AI is allowed to learn them as learning data. As a result, when the word representation of the flow pattern is input, the AI may calculate and output the three-dimensional shape of the structure that realizes the flow pattern.

Functions and effects of the learning method of the structure shape according to this embodiment are described. For example, at step S120, even if it is attempted to allow the AI to directly learn the three-dimensional shape of the structure and the flow pattern as the learning data, learning is actually difficult. This is because the flow pattern itself is too complicated as a data structure. On the other hand, this embodiment is characterized in that the word representation of the streamline structure of the flow pattern is temporarily performed, and this word representation and the three-dimensional shape of the structure are made the learning data. That is, by representing a complicated flow pattern by a finite character string such as the COT representation, the data structure becomes simple, so that the learning by the AI becomes possible. As described above, according to this embodiment, it is possible to obtain the optimum structure shape for controlling the flow of the fluid by using the learning by the AI.

### Sixth Embodiment

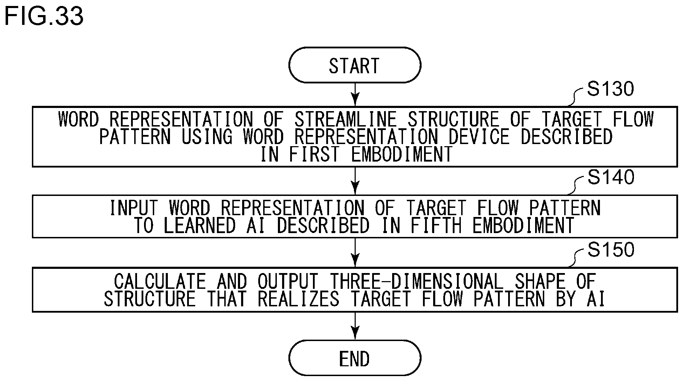

A sixth embodiment of the present invention is a shape designing method of a structure. Fig. 33 illustrates a flow of a structure designing method according to the sixth embodiment. This method includes step S130 of performing word representation of a streamline structure of a target flow pattern using the word representation device according to the first embodiment, step S140 of inputting the word representation of the target flow pattern to the learned AI according to the fifth embodiment, and step S150 of calculating and outputting a three-dimensional shape of a structure that realizes the target flow pattern using the learned AI.

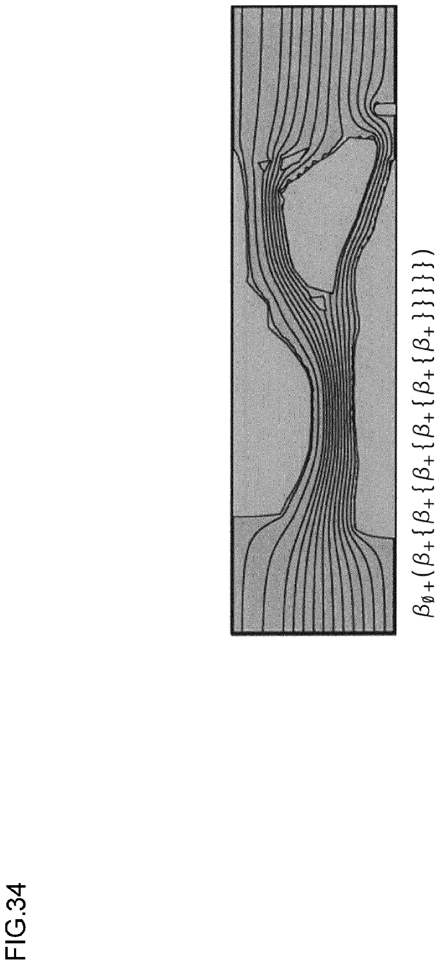

At step S130, this method performs word representation of the stream structure of the target flow pattern using the word representation device according to the first embodiment. At that time, for example, a user may draw a desired flow pattern on a topographic map of a river using drawing software, and allow a word representation device 1 in Fig. 22, for example, to read the flow pattern, thereby obtaining a COT representation. Fig. 34 illustrates the desired flow pattern drawn by the user for a topographic diagram in Fig. 31 and the COT representation thereof.

At step S140, this method inputs the word representation (COT representation) of the target flow pattern created at step S130 to the learned AI described in the fifth embodiment.

At step S150, this method outputs, from the learned AI, an optimum shape of the structure for realizing the word representation from the word representation of the target flow pattern input at step S140 on the basis of a learning result at step S120. On the basis of this output result, by forming the target structure by, for example, dredging, sediment throwing, installation of wave absorbing blocks and the like, a current three-dimensional shape of the river is corrected, and a desired flow pattern may be obtained.

According to this embodiment, by inputting the target flow pattern, a specific three-dimensional shape of the structure that realizes the flow pattern may be obtained.

Note that, for example, in a case where the target structure is formed by installing a large number of fish reefs on the basis of the output result from the AI, the shape of the structure might have an error with respect to an ideal shape output by the AI. In such a case, the flow pattern realized by the formed structure may be drawn by simulation, and the word representation of the streamline structure may be performed. Then, this word representation may be compared with the word representation of the streamline structure of the flow pattern obtained from the ideal shape output by the AI. As a result of this comparison, when the word representation of the desired flow pattern is not realized, the desired flow pattern may be drawn in a shape drawing at that time, and the processing at steps S130 to S150 may be performed again. By repeatedly performing such confirmation and correction work as necessary, it is possible to design a structure with higher accuracy.

The method described above is not limited to the control of the flow of the river. The desired shape of the structure may be output by allowing the AI to learn the relationship between the shape of the structure and the COT representation of the fluid flow around the structure and then inputting the COT representation of the target fluid flow pattern to the AI. This may also be applied to, for example, design of an engine with high combustion efficiency, design of a screw shape with high thrust and the like.

It is described above on the basis of some embodiments of the present invention. Those skilled in the art understand that these embodiments are exemplary and that various variations and modifications are possible within claims of the present invention, and that such variations and modifications are also within claims of the present invention. Therefore, the descriptions and drawings in this specification should be treated as exemplary rather than limiting.

### Variation 1

The word representation device of the first or second embodiment may be provided with an image acquisition unit (for example, a camera and the like) that acquires an image of a given flow pattern. The image acquired by the image acquisition unit is transmitted to a word representation generator, and a word representation is given by the above-described processing. According to this variation, when the image of the flow pattern is given, it is possible to capture the same to obtain the word representation.

### Variation 2

The word representation device of the first or second embodiment may be provided with an image recognition unit in addition to the image acquisition unit of the variation 1. An image acquired by the image acquisition unit is transmitted to the image recognition unit, and a fluid structure in the image is recognized. The recognized fluid structure is transmitted to a word representation unit and a word representation is provided by the processing described above. According to this variation, when an image of a flow pattern is given, an accurate word representation may be obtained by recognizing the fluid structure in the image.

The variation has functions and effects similar to those of the above-described embodiment.

Any combination of the above-described embodiments and variations is also useful as the embodiment of the present invention. A new embodiment generated by the combination has effects of each of the combined embodiments and variations.

### [INDUSTRIAL APPLICABILITY]

The present invention may be used for analysis of medical images, design of buildings and industrial products, weather prediction, disaster countermeasures by fluid analysis of rivers and sea surfaces, and fishery.

### [REFERENCE SIGNS LIST]

1 Word representation device, 2 Word representation device, 10 Storage, 21 Route determination means, 22 Tree representation forming means, 23 COT representation generation means, 24 Combinatorial structure extraction means, S1 Step of determining root, S2 Step of forming tree representation, S3 Step of generating COT representation, S110 Step of performing word representation of streamline structure of flow pattern generated around structure in fluid, S120 Step of learning with AI such that three-dimensional shape of structure is output by using word representation as input, S130 Step of performing word representation of the streamline structure of target flow pattern using device according to first embodiment, S140 Step of inputting word representation of target flow pattern to learned AI according to fifth embodiment., S150 Step of calculating three-dimensional shape of structure that realizes target flow pattern using AI and outputting

## Claims

1. A word representation device that performs word representation of a streamline structure of a flow pattern in a two-dimensional domain, the device comprising:
a storage; and
a word representation generator, wherein
the storage stores a correspondence relationship between each streamline structure and a character of each streamline structure regarding a plurality of streamline structures forming the flow pattern,
the word representation generator is provided with a root determination means, a tree representation forming means, and a COT representation generation means,
the root determination means determines a root of a given flow pattern,
the tree representation forming means forms a tree representation of the given flow pattern by repeatedly executing processing of extracting a streamline structure of the given flow pattern, assigning a character to the extracted streamline structure on the basis of the correspondence relationship stored in the storage, and deleting the extracted streamline structure from an innermost portion of the flow pattern until reaching the root, and
the COT representation generation means converts the tree representation formed by the tree representation forming means to a COT representation to generate a word representation of the given flow pattern.

2. The word representation device according to claim 1, wherein among the streamline structures forming the flow pattern, basic structures are σ_{ϕ±}, σ_{ϕ~±0}, σ_{ϕ~±±}, σ_{ϕ~±}∓, β_{ϕ±}, and β_{ϕ2}.

3. The word representation device according to claim 1 or 2, wherein among the streamline structures forming the flow pattern, two-dimensional structures are b~± and b_{±}.

4. The word representation device according to any one of claims 1 to 3, wherein among the streamline structures forming the flow pattern, zero-dimensional point structures are σ_{~±0}, σ_{~±±}, and σ_{~±}∓.

5. The word representation device according to any one of claims 1 to 4, wherein among the streamline structures forming the flow pattern, one-dimensional structures are p_{~±}, p_{±}, a_{±}, q_{±}, b_{±±}, b_{±}∓, β_{±}, c_{±}, c_{2±}, a₂, γ_{ϕ~±}, γ_{~±±}, a_{~±}, and q_{~±}.

6. The word representation device according to any one of claims 1 to 5, wherein
the word representation generator is further provided with a combinatorial structure extraction means, and
the combinatorial structure extraction means generates a word representation having a one-to-one correspondence of the given flow pattern by extracting a combinatorial structure from the given flow pattern.

7. A word representation method of performing word representation of a streamline structure of a flow pattern in a two-dimensional domain executed by a computer provided with a storage and a word representation generator,
the storage storing a correspondence relationship between each streamline structure and a character of each streamline structure regarding a plurality of streamline structures forming the flow pattern, and
the word representation generator executing a root determination step, a tree representation forming step, and a COT representation generation step, wherein
the root determination step determines a root of a given flow pattern,
the tree representation forming step forms a tree representation of the given flow pattern by repeatedly executing processing of extracting a streamline structure of the given flow pattern, assigning a character to the extracted streamline structure on the basis of the correspondence relationship stored in the storage, and deleting the extracted streamline structure from an innermost portion of the flow pattern until reaching the root, and
the COT representation generation step converts the tree representation formed at the tree representation forming step to a COT representation to generate a word representation of the given flow pattern.

8. A program that allows a computer provided with a storage and a word representation generator to execute processing,
the storage storing a correspondence relationship between each streamline structure and a character of each streamline structure regarding a plurality of streamline structures forming the flow pattern,
the program structured to allow the word representation generator to execute:
a root determination step of determining a root of a given flow pattern;
a tree representation forming step of forming a tree representation of the given flow pattern by repeatedly executing processing of extracting a streamline structure of the given flow pattern, assigning a character to the extracted streamline structure on the basis of the correspondence relationship stored in the storage, and deleting the extracted streamline structure from an innermost portion of the flow pattern until reaching the root; and
a COT representation generation step of converting the tree representation formed at the tree representation forming step to a COT representation to generate a word representation of the given flow pattern.

9. A method of learning a shape of a structure in a fluid in a two-dimensional domain, the method comprising:
performing word representation of a streamline structure of a flow pattern generated around a structure in a fluid by using the word representation device according to claim 1; and
learning by AI such that a three-dimensional shape of the structure is output by using the word representation as an input.

10. A structure designing method of designing a structure in a fluid in a two-dimensional domain, the method comprising:
performing word representation of a streamline structure of a target flow pattern using the word representation device according to claim 1;
inputting a word representation of the target flow pattern to the learned AI according to claim 9; and
calculating and outputting a three-dimensional shape of a structure that realizes the target flow pattern using the learned AI.
